# EUROPEAN PATENT APPLICATION

(11) **EP 3 900 747 A1**
(43) Date of publication of application: **27.10.2021**
(21) Application number: 20382393.5
(22) Date of filing: 12.05.2020
(51) Int. Cl.: A61L 2/20

(54) **APPARATUS AND METHOD FOR SANITIZING ITEMS WITH OZONE GAS**

(30) Priority: 21.04.2020 EP 20382326
(71) Applicant: Jeanología, S.L., 46980 Paterna, Valencia (ES)
(72) Inventor: SILLA VIDAL, Enrique, 46980 Paterna (Valencia) (ES); ALBERT REVERT, Vicente, 46980 Paterna (Valencia) (ES); FUSTER FUSTER, Vicente, 46980 Paterna (Valencia) (ES); GARCIA CABRERA, Jesus, 46980 Paterna (Valencia) (ES)
(74) Representative: González Poveda, Sara

(57) **Abstract**

Apparatus and method for sanitizing items with ozone, the apparatus comprising a disinfection chamber which has an entrance door and an exit door, an entrance antechamber that has an external entrance, an exit antechamber that has an external exit door a motorized item transport system an ozone generator, an ozone destroy system, a gas extraction system, sensing means, a control and power system, the doors being automatic. The apparatus optionally comprises a heating system and/or second heating system and/or a third heating system. The method comprises using the apparatus and components therein. A non-transitory computer readable medium may contain instructions for operating the apparatus for implementing the method.

## Description

### Technical Field

The present invention refers to the technical field of sanitizing (disinfecting) items, such as for example wearables or clothes or apparel, or dresses, or accessories, or personal protection equipment (PPE) or shoes or home textiles (e.g. linens, towels, bed sheets, etc.) using ozone gas. Sanitizing items can also be disinfecting items. More specifically the present application describes apparatuses and methods for sanitizing (or disinfecting) wearables using ozone gas. In the context of the present application an apparatus described herein may be also considered as being a device or a system, or an assembly of devices or subsystems, or a machine, or an assembly of machines. A wearable should be understood as being any type of item that may be worn on the human body, such as for example an article of clothing, shoes, a clothing accessory, a personal protective equipment item, a sports wearable o handheld item and the like. Alternatively, the apparatus and method described herein may be used for sanitizing any item that fits within the apparatus. It is of particular interest using the present invention for disinfecting the wearables or items from pathogens such as biological pathogens, bacteria, germs and viruses, for example coronavirus, which may be present on the wearables or items as a result of the wearable's location and/or use, such as for example the wearable's presence and use in a hospital with infected patients or workers, or within an apparel shop wherein some of the workers or clients carrying a pathogen may contaminate said wearables, or more generally by the use or the proximity of the wearable to a patient or source that releases said pathogen. Said items or wearables upon efficiently disinfected or sanitized may be used or re-used safely, and this has tremendous health and economic advantages for their users and for the society. Sanitizing refers to fully or partially removing or killing or eliminating or neutralizing said pathogens from said items or wearables, or may alternatively or in parallel relate to eliminating odors from the wearables, or may relate to treating the items or wearables or linens with ozone as a part of a safety and quality control protocol applied in the places where said items are produced or processed or stored or handled or used.

### Prior art.

The 2019-20 coronavirus pandemic which has been caused by the ongoing spread of the SARS-COV-2 virus that causes to humans the COVID-19 disease, has a devastating impact on the public health and the economy at a global scale. Efforts to fight this pandemic as well as other epidemics include the adaptation and application of practices related to disinfecting everyday use items and surfaces. It is well known that a human carrier of biological pathogens, such as viruses or bacteria, may pass said pathogens to a surface, and often said pathogens may stay on said surface for several hours or days without losing their ability to infect others who will come into contact with the surface and obtain therefrom the pathogens. This is often the case with the surfaces of wearables such as for example the surfaces of apparel or personal protective equipment such as googles, scrubs and protection masks, used by doctors, nurses and patients in hospitals having patients being infected with pathogens. It is highly desirable disinfecting said surfaces, items and wearables as often as possible, and preferably every time before they are being used or worn. By disinfecting them, meaning eliminating or removing or neutralizing any of the pathogens on them, said items or wearables are safe to use or re-use. Likewise, in a factory or logistics sender or store were items such as apparel or shoes are made or processed or stored or sold, it is very useful that a treatment is applied to said items so that said items are disinfected or sanitized.

There exist several known methods for killing biological pathogens. One of them concerns the use of ozone gas. It is well known that ozone gas can kill bacteria and viruses. For this reason, ozone gas is often used for disinfecting water, such as wastewater. In fact, often, disinfecting wastewater with ozone is a preferred technique over others because comparatively the former is considered to be eco-friendlier and more cost effective.

In view of said proven use of ozone gas to disinfect and eliminate biological pathogens, and in view of the proven fact that epidemics such as the 2019-20 pandemic have caused a great need for the widespread adaptation of techniques for disinfecting everyday items such as apparel or shows or wearables for the everyday use and re-use of said items, the problem is how to best implement ozone gas for disinfecting and thusly safely reusing everyday items such as wearables and personal protective equipment, especially when the demand for safely using and reusing said items is temporarily increased and/or spiking.

### Description of the invention

It is a first object of the invention to provide an apparatus for disinfecting items, particularly wearables. The apparatus may also be considered as being a machine or a system or a device. It is contemplated that the apparatus is adapted to the need of the industry related to the fabrication, processing or sell of wearables and apparel. To this end the apparatus has certain features that make it easy to install and use, safe to use, and result to achieving high efficiency, efficacy and productivity when using the apparatus. Advantageously the apparatus is compact and it is contemplated that its use reduce the costs associated with sanitizing objects, and may also result to implementing a safety protocol.

It is a second object of the invention to provide methods for disinfecting items, particularly wearables or clothes or shoes or apparel, using the apparatus related to the first object.

A further object of the invention is a software or a non-transitory computer readable medium having instructions to apply (implement) a method that is according to the second object of the invention, preferably using an apparatus and a computer therein that are according the first object of the invention.

The efficient use of ozone gas for disinfecting items or apparel or garments or shoes or linens or wearables and/or other everyday items requires having machines, meaning devices or systems or apparatuses, that are easy and safe to use, they are compact and/or can be moved and installed to the place of use, and have a proper form so they can be used in establishments wherein there is a high demand for disinfecting or sanitizing high volumes or numbers of items. Non limiting examples of such establishments are healthcare establishments, stores such as apparel and clothing stores, factories or other commercial buildings and installations, places wherein food is produced or processed, public service buildings, logistic centers, storage facilities, and the like. For example, in a healthcare establishment such as in a hospital it is required to use ozone gas for disinfecting personal protection equipment such as face masks, scrubs and googles for safely reusing said equipment as often as possible and/or as often as is required depending on the viral load in said hospitals at any given time. Likewise, in a clothing store or a clothing storage and logistics center, and especially in times of a pandemic, it is desirable that the cloths or items therein are disinfected every time before or after they are used or worn or tried by the clients or send to the clients or to the users of such items.

Therefore, a technical problem is how to design machines, systems, devices for frequently or daily disinfecting items for using or reusing said items, and especially for doing said disinfection close, or at the place wherein, said items are made or processed or sold or used. This technical problem entails the additional problems of how to make these machines to be safe, easy to use, cost-effective, compact, easily movable, energy efficient, and easy to produce at a mass scale and at a comparatively low cost. The present invention addresses all the aforementioned problems.

The invention in its first aspect concerns an apparatus for sanitizing items, the apparatus comprising:
- a disinfection chamber which has an entrance door and an exit door and is configured so that the items to be sanitized enter within it via the entrance door and exit from the disinfection chamber via the exit door, the entrance door and the exit door being automatic;
- an entrance antechamber that has an external entrance door and is adjacent to the disinfection chamber and the entrance door thereat, and is configured and arranged so that the items before entering the disinfection chamber pass via the external entrance door and through the entrance antechamber, the external entrance door being automatic;
- an exit antechamber that has an external exit door and is adjacent to the disinfection chamber and the exit door thereat, and is configured and arranged so that the items upon exiting the disinfection chamber pass through the exit antechamber and exit therefrom via the external exit door;
- a motorized item transport system that when activated, when the apparatus is operated, wherein said motorized item transport system is configured to convey the items so that the items successively pass through the external entrance door and the entrance antechamber, enter and pass through the disinfection chamber and exit from the latter, and pass through the exit antechamber and exit therefrom via the external exit door;
- an ozone generator connected to the disinfection chamber and configured to generate ozone gas and supply the latter to within (the interior of) the disinfection chamber when the apparatus is operated;
- an ozone destroy system connected to the disinfection chamber and comprising an end exhaust and configured to receive from within (the interior of) the disinfection chamber ozone gas and to convert said ozone gas to a non-toxic gaseous product, and to preferably release said non-toxic gaseous product via the end exhaust, when the apparatus is operated;
- a gas extraction system that is configured to extract gases from within the exit antechamber and the entrance antechamber when the apparatus is operated;
- sensing means configured for sensing at least one operational parameter and generating and transmit a corresponding sensing signal when the apparatus is operated;
- a control and power system for controlling operating the apparatus, the control and power system comprising an electrical panel for controlling powering the apparatus, a computer and user command means configured for receiving operation commands from a user, and the control and power system is configured to control the operation of the apparatus according to the operation commands, and is configured to receive the sensing signal and inhibit the operation of the apparatus when the sensing signal does not correspond to a safety condition.

The apparatus may also be considered as being a machine or a system or a device or an assembly of devices or subsystems. The ozone destroy system ensures that the exhaust product of the device is non-toxic and poses no harm to the users. The sensing means, which in a non-limiting preferable example comprise a first ozone gas sensor installed within the disinfection chamber and configured to measure the ozone concentration in gas, and a second ozone gas sensor installed outside the chamber and configured to sense whether any undesired leakage of ozone takes place, and a sensor that detects whether any of the doors is closed or open, solves the technical problem on how to know whether the operational parameters are within any specifications dictated by the user or the device manufacturer so that the disinfection is efficient and safe. The power and control unit and in particular the computer, said computer preferably being a programmable logic controller (PLC), provide for an accurate and well controlled operation of the device, and preferably coordinate the operation of the electronic or electrical or electromechanical components therein when the apparatus is used/operated. The user control means, which in an example comprises a touch screen connected to the PLC, allows for an easy and/or advanced use of the system by the users. In a non-limiting example, the safety condition is that the ozone concentration outside the disinfection chamber and outside the entrance antechamber and the exit antechamber is below 0.05 ppm as measured by an ozone gas sensor thereabout.

Optionally and preferably the ozone generator is configured to generate ozone gas at a rate of between 1 grams of ozone per hour to 100 grams of ozone per hour, and preferably of between 50 grams of ozone per hour to 60 grams of ozone per hour; at lower rates the ozone gas concentration may not be sufficient for achieving a sufficient degree of disinfection, and at higher rates the ozone gas concentration may be too high causing deterioration of the items to be disinfected.

As mentioned, the exit door, the entrance door, the external exit door and the internal exit door are automatic, and this means that they are configured to be controllable by the power and control system, and each of said doors is configured to open and close by a respective electromechanical actuation mechanism, as is for example a motor, that is a part of or is attached to said door. Said actuation mechanism is connected to and controlled by the power and control system which is configured to activate and/or actuate said mechanism. Therefore, preferably the exit door comprises an exit door actuation mechanism (motor), the entrance door comprises an entrance door actuation mechanism, the external exit door comprises an external exit door actuation mechanism, and the external entrance door comprises an external entrance door actuation mechanism, and each of said actuation mechanisms may be independently connected to and be controllable by the power and control system. Furthermore, each door optionally is a two leaf door, and in that case the leafs of each door are optionally slidable (can slide between an open position and a closed position) or foldable (can be folded between a door-open position and a door-closed position).

Further preferably the invention in its first aspect concerns an apparatus for sanitizing items, such as for example wearables, apparel or shoes, the apparatus comprising:
- a disinfection chamber which has an entrance door and an exit door and is configured so that the items to be sanitized enter therein, meaning within it, via the entrance door, and exit therefrom, meaning from within the disinfection chamber, via the exit door, the entrance door and the exit door being automatic;
- a tubing system that has an ozone inlet, an ozone outlet, an exhaust inlet, an exhaust outlet and an end exhaust, wherein the tubing system is connected to the disinfection chamber via the ozone outlet and the exhaust inlet, and is configured to, during an operation of the apparatus by a user, supply to the disinfection chamber ozone gas via the ozone outlet, and to receive from the disinfection chamber ozone gas via the exhaust inlet;
- an entrance antechamber that has an external entrance door and is adjacent to the disinfection chamber and to the entrance door thereat, and is configured and arranged so that the items before entering the disinfection chamber pass via the external entrance door and through the entrance antechamber, the external entrance door being automatic;
- an exit antechamber that has an external exit door and is adjacent to the disinfection chamber and to the exit door thereat, and is configured and arranged so that the items upon exiting the disinfection chamber pass through the exit antechamber and exit therefrom via the external exit door, the external exit door being automatic;
- a motorized item transport system that is configured to convey the items so that, when the apparatus is operated, the items successively pass through the external entrance door and the entrance antechamber, enter and pass through the disinfection chamber and exit from the latter, and pass through the exit antechamber and exit therefrom via the external exit door;
- an ozone generator connected to the tubing system and configured to generate ozone gas and supply via the ozone inlet said ozone gas to the tubing system when the apparatus is operated, the ozone generator optionally and preferably being configured to generate ozone gas and to supply said ozone gas to the disinfection chamber;
- an ozone destroy system that is connected to the tubing system and is configured to receive from the latter via the exhaust outlet ozone gas that has been received by the tubing system via the exhaust inlet, and to convert said ozone gas to a non-toxic gaseous product, and to supply said non-toxic gaseous product to the end exhaust;
- a gas extraction system that is configured to extract gases from within the exit antechamber and from the entrance antechamber when the apparatus is operated;
- sensing means configured for sensing at least one operational parameter and for generating and transmit a corresponding sensing signal when the apparatus is operated;
- a control and power system for controlling operating the apparatus, the control and power system comprising an electrical panel for controlling powering the apparatus, a computer and user command means configured for receiving operation commands from a user, the computer optionally preferably being a programmable logic controller, the user command means optionally and preferably comprising a touchscreen, and the control and power system is configured to control the operation of the apparatus according to the operation commands, and is configured to receive the sensing signal and inhibit the operation of the apparatus when the sensing signal does not correspond to a safety condition.

When herein it is described that an item passes via a door (any of the doors), it means that item passes when the door is open thusly allowing the item to pass there through.

As described further above, optionally and preferably the ozone generator of the apparatus is configured to generate ozone gas at a rate of between 1 grams of ozone per hour to 100 grams of ozone per hour, and preferably of between 50 grams of ozone per hour to 60 grams of ozone per hour.

Optionally and preferably the apparatus comprises a heating system that is configured to increase the temperature inside the disinfection chamber. when the ozone is supplied to the disinfection chamber when the gaseous atmosphere (gas environment) inside said chamber is hot, then the items inside said chamber are being sanitized better and faster. It is contemplated by the inventors of the present invention that when the heating system causes the temperature inside the disinfection chamber to increase, the supplied ozone neutralizers faster and more efficient any pathogens on the items inside the disinfection chamber. For achieving better, easier and more accurate control and operation of the heating system, optionally and preferably the heating system is connected to and configured to be controllable (controlled) by the control and power system, and the latter is configured to control the heating system.

Optionally and preferably the heating system comprises a heating element installed (located) inside the disinfection chamber or inside the tubing system that is connected to and communicates with said disinfection chamber. The heating element can for example be an electrical resistance or a heating (IR) lamp or a heat exchanger that produces heat when supplied with electrical current. Optionally and preferably the heating element comprises between 1 and 10 electrical resistances and more preferably between 1 and 4 electrical resistances that preferably is configured to operate each electrical resistance at a nominal power in the range of 1 kW to 10 kW, and more preferably in the range of 1 kW to 3 kW. At said nominal powers, the temperature in the disinfection chamber can be increased quickly when the heating system is operated. Likewise, optionally and preferably the heating system comprises a temperature sensor that is configured to measure the temperature inside the disinfection chamber or inside the tubing system. Preferably the measurement by the temperature sensor us used as feedback for/when operating/activating/deactivating/powering-on/powering-off the heating element. In a non-limiting example, the temperature sensor is a Pt100 sensor.

Preferably the heating system heats the gaseous atmosphere inside the disinfection chamber. Said gaseous atmosphere is the mixture of any gases inside the disinfection chamber. Therefore, said gaseous atmosphere may be the air inside the disinfection chamber. Heating said gaseous atmosphere results to heating the items (wearables) inside the disinfection chamber. For heating said gaseous atmosphere, optionally and preferably the heating system comprises an auxiliary suction pump that is configured to force any gas from inside the disinfection chamber to flow by the heating element and to subsequently flow inside the disinfection chamber. The auxiliary suction pump may be technically similar to the suction pump that is described further below. Likewise, optionally and preferably, the tubing system comprises a tubing circuit, and the heating element and the auxiliary pump are installed (located, connected to) at said tubing circuit, and the tubing system and heating system, when the latter is operated, are configured to extract (any) gas from the disinfection chamber via said auxiliary suction pump, pass said gas via the tubing circuit and by the heating element thereat, heat said gas with the heating element, and reintroduce the thusly heated gas into the disinfection chamber; with the aforementioned optional configuration, the heating system can heat the gaseous atmosphere (any gas) of the disinfection chamber. Preferably the tubing circuit is separate from the rest of the tubing system so that it is avoided heating the ozone gas before the latter enters the disinfection chamberFor this reason, optionally and preferably, the tubing circuit comprises a tubing circuit inlet connected (installed on) to the disinfection chamber, a tubing circuit outlet connected to (installed on) the disinfection chamber, and the auxiliary suction pump when operated is configured to extract gas from the disinfection chamber via said tubing circuit inlet, and reintroduce the gas intro disinfection chamber via said tubing circuit outlet. Preferably, the temperature inside the disinfection chamber is the temperature of a gaseous atmosphere that is inside said disinfection chamber, or is the temperature of a gaseous atmosphere that is inside the tubing circuit connected to said disinfection chamber, or is the temperature of any part of said gaseous atmosphere, and further preferably is the temperature measured by the temperature sensor. It is contemplated that in the apparatus (device, system) the ozone gas sanitizes (disinfects) better the items (e.g. the wearables, clothes) when the temperature of the latter is approximately of between 50°C and 70°C. For this reason, the best desired disinfection temperature (desired sanitization temperature) of the gaseous atmosphere is of between 80°C and 100°C, because it must be considered that in practice during a sanitization/disinfection cycle in some cases there will be a difference between the temperature of the items and the temperature of the gaseous atmosphere surrounding said items. Therefore, optionally and preferably the heating system is configured to increase the temperature inside the disinfection chamber to a desired disinfection temperature, said desired disinfection temperature preferably being of between 25°C and 100°C, and more preferably of between 40°C to 90°C, and most preferably of between 60°C and 90°C.

According to the above, optionally and preferably in the apparatus (system) of the first aspect of the invention the tubing system further comprises a tubing circuit that has a circuit inlet and a circuit outlet, said tubing circuit being connected to the disinfection chamber via the circuit inlet and the circuit outlet, and moreover the device further comprises a heating system that when operated is configured to increase a temperature inside the disinfection chamber, the heating system comprising a heating element, an auxiliary suction pump, and a temperature sensor, the heating element when operated being configured to heat inside the tubing circuit, the auxiliary suction pump when operated being configured to force any gas from inside the disinfection chamber to successively exit said disinfection chamber via said circuit inlet, flow inside said tubing circuit, and reenter the disinfection chamber via the circuit outlet.

Optionally and preferably, a method or an operation command related to operating the system (device), comprises operating or activating or deactivating the heating system and one or more components of the heating system, during any of the following and combinations thereof:
- before injecting (supplying) ozone to the disinfection chamber
- when injecting ozone to the disinfection chamber
- after injecting ozone to the disinfection chamber.

Preferably the heating system is not being activated (i.e. is not switched on) when injecting ozone to the disinfection chamber.

The ventilation system that is described further down, may contribute to homogenizing the temperature inside the disinfection chamber. For sanitizing some items with the apparatus, for example when applying a sanitization cycle, the heating system heats for a total period of time that can also be called a heating duration.

Optionally and preferably the apparatus comprises a second heating system that is configured to increase the temperature inside the entrance antechamber. It is contemplated by the inventors of the present invention that when the heating system causes the temperature inside the entrance antechamber to increase, the supplied ozone neutralizers faster and more efficient any pathogens on the items inside the disinfection chamber. For achieving better, easier and more accurate control and operation of the second heating system, optionally and preferably the heating system is connected to and configured to be controllable (controlled) by the control and power system, and the latter is configured to control the second heating system.

Optionally and preferably the second heating system comprises a second heating element (or a plurality of second heating elements) installed (located) inside the entrance antechamber or inside the tubing system that is connected to and communicates with said entrance antechamber. The second heating element can for example be an electrical resistance or a heating (IR) lamp or a heat exchanger that produces heat when supplied with electrical current. Optionally and preferably the second heating element comprises between 1 and 10 electrical resistance and more preferably between 1 and 4 electrical resistance that preferably is configured to operate each electrical resistance at a nominal power in the range of 1 kW to 10 kW, and more preferably in the range of 1 kW to 3 kW. At said nominal powers, the temperature in the entrance antechamber can be increased quickly when the heating system is operated. Likewise, optionally and preferably the heating system comprises a second temperature sensor that is configured to measure the temperature inside the entrance antechamber or inside the tubing system. Preferably the measurement by the second temperature sensor is used as feedback for/when operating/activating/deactivating/powering-on/powering-off the heating element. In a non-limiting example, the temperature sensor is a Pt100 sensor.

Preferably the heating system heats the gaseous atmosphere inside the entrance antechamber. Said gaseous atmosphere is the mixture of any gases inside the disinfection chamber. Therefore, said gaseous atmosphere may be the air inside the entrance antechamber. Heating said gaseous atmosphere results to heating the items (wearables) inside the entrance antechamber. For heating said gaseous atmosphere, optionally and preferably the heating system comprises an second auxiliary suction pump that is configured to force any gas from inside the entrance antechamber to flow by the second heating element and to subsequently flow inside the entrance antechamber. The auxiliary suction pump may be technically similar to the suction pump that is described further below. Likewise, optionally and preferably, the tubing system comprises a second tubing circuit, and the second heating element and the second auxiliary pump are installed (located, connected to) at said second tubing circuit, and the second tubing system and second heating system, when the latter is operated, are configured to extract (any) gas from the entrance antechamber via said second auxiliary suction pump, pass said gas via the second tubing circuit and by the second heating element thereat, heat said gas with the second heating element, and reintroduce the thusly heated gas into the entrance antechamber; with the aforementioned optional configuration, the second heating system can heat the gaseous atmosphere (any gas) of the entrance antechamber. Preferably the second tubing circuit is separate from the rest of the tubing system so that the overall system is easier and simpler to operate and prepare. For this reason, optionally and preferably, the second tubing circuit comprises a second tubing circuit inlet connected (installed on) to the entrance antechamber, a second tubing circuit outlet connected to (installed on) the entrance antechamber, and the second auxiliary suction pump when operated is configured to extract gas from the entrance antechamber via said second tubing circuit inlet, and reintroduce the gas into the entrance antechamber via said second tubing circuit outlet. Preferably, the temperature inside the entrance antechamber is the temperature of a gaseous atmosphere that is inside said entrance antechamber, or is the temperature of a gaseous atmosphere that is inside the second tubing circuit connected to said entrance antechamber, or is the temperature of any part of said gaseous atmosphere, and further preferably is the temperature measured by the second temperature sensor. It is contemplated that in the apparatus (device, system) the ozone gas sanitizes (disinfects) better the items (e.g. the wearables, clothes) when the temperature of the latter is approximately of between 50°C and 70°C. For this reason, the best desired disinfection temperature (desired sanitization temperature) of the gaseous atmosphere is of between 80°C and 100°C, because it must be considered that in practice during a sanitization/disinfection cycle in some cases there will be a difference between the temperature of the items and the temperature of the gaseous atmosphere surrounding said items. Therefore, optionally and preferably the second heating system is configured to increase the temperature inside the entrance antechamber to a second desired disinfection temperature, said second desired disinfection temperature preferably being of between 25°C and 100°C, and more preferably of between 40°C to 90°C, and most preferably of between 60°C and 90°C.

According to the above, optionally and preferably in the apparatus (system) of the first aspect of the invention the tubing system further comprises a second tubing circuit that has a second circuit inlet and a second circuit outlet, said second tubing circuit being connected to the entrance antechamber via the second circuit inlet and the second circuit outlet, and moreover the device further comprises a second heating system that when operated is configured to increase a temperature inside the entrance antechamber, the second heating system comprising a second heating element (or a plurality of second heating elements), a second auxiliary suction pump, and a second temperature sensor, the second heating element when operated being configured to heat inside the second tubing circuit, the second auxiliary suction pump when operated being configured to force any gas from inside the entrance antechamber to successively exit said entrance antechamber via said second circuit inlet, flow inside said second tubing circuit, and reenter the entrance antechamber via the second circuit outlet.

Optionally and preferably, a method or an operation command related to operating the system (device), comprises operating or activating or deactivating the second heating system and one or more components of the second heating system, during any of the following and combinations thereof:
- before injecting (supplying) ozone to the disinfection chamber
- when injecting ozone to the disinfection chamber
- after injecting ozone to the disinfection chamber.

Preferably the second heating system is not being activated (i.e. is not switched on) when injecting ozone to the disinfection chamber.

For sanitizing some items with the apparatus, for example when applying a sanitization cycle, the second heating system heats for a total period of time that can also be called a second heating duration.

Optionally and preferably the apparatus comprises a third heating system that is configured to increase the temperature inside the exit antechamber. It is contemplated by the inventors of the present invention that when the heating system causes the temperature inside the exit antechamber to increase, the supplied ozone neutralizers faster and more efficient any pathogens on the items inside the disinfection chamber. For achieving better, easier and more accurate control and operation of the third heating system, optionally and preferably the heating system is connected to and configured to be controllable (controlled) by the control and power system, and the latter is configured to control the third heating system.

Optionally and preferably the third heating system comprises a third heating element (or a plurality of third heating elements) installed (located) inside the exit antechamber or inside the tubing system that is connected to and communicates with said exit antechamber. The third heating element can for example be an electrical resistance or a heating (IR) lamp or a heat exchanger that produces heat when supplied with electrical current. Optionally and preferably the third heating element comprises between 1 and 10 electrical resistance and more preferably between 1 and 4 electrical resistance that preferably is configured to operate each electrical resistance at a nominal power in the range of 1 kW to 10 kW, and more preferably in the range of 1 kW to 3 kW. At said nominal powers, the temperature in the exit antechamber can be increased quickly when the heating system is operated. Likewise, optionally and preferably the heating system comprises a third temperature sensor that is configured to measure the temperature inside the exit antechamber or inside the tubing system. Preferably the measurement by the third temperature sensor is used as feedback for/when operating/activating/deactivating/powering-on/powering-off the heating element. In a non-limiting example, the temperature sensor is a Pt100 sensor.

Preferably the heating system heats the gaseous atmosphere inside the exit antechamber. Said gaseous atmosphere is the mixture of any gases inside the disinfection chamber. Therefore, said gaseous atmosphere may be the air inside the exit antechamber. Heating said gaseous atmosphere results to heating the items (wearables) inside the exit antechamber. For heating said gaseous atmosphere, optionally and preferably the heating system comprises an third auxiliary suction pump that is configured to force any gas from inside the exit antechamber to flow by the third heating element and to subsequently flow inside the exit antechamber. The auxiliary suction pump may be technically similar to the suction pump that is described further below. Likewise, optionally and preferably, the tubing system comprises a third tubing circuit, and the third heating element and the third auxiliary pump are installed (located, connected to) at said third tubing circuit, and the third tubing system and third heating system, when the latter is operated, are configured to extract (any) gas from the exit antechamber via said third auxiliary suction pump, pass said gas via the third tubing circuit and by the third heating element thereat, heat said gas with the third heating element, and reintroduce the thusly heated gas into the exit antechamber; with the aforementioned optional configuration, the third heating system can heat the gaseous atmosphere (any gas) of the exit antechamber. Preferably the third tubing circuit is separate from the rest of the tubing system so that the overall system is easier and simpler to operate and prepare. For this reason, optionally and preferably, the third tubing circuit comprises a third tubing circuit inlet connected (installed on) to the exit antechamber, a third tubing circuit outlet connected to (installed on) the exit antechamber, and the third auxiliary suction pump when operated is configured to extract gas from the exit antechamber via said third tubing circuit inlet, and reintroduce the gas into the exit antechamber via said third tubing circuit outlet. Preferably, the temperature inside the exit antechamber is the temperature of a gaseous atmosphere that is inside said exit antechamber, or is the temperature of a gaseous atmosphere that is inside the third tubing circuit connected to said exit antechamber, or is the temperature of any part of said gaseous atmosphere, and further preferably is the temperature measured by the third temperature sensor. It is contemplated that in the apparatus (device, system) the ozone gas sanitizes (disinfects) better the items (e.g. the wearables, clothes) when the temperature of the latter is approximately of between 50°C and 70°C. For this reason, the best desired disinfection temperature (desired sanitization temperature) of the gaseous atmosphere is of between 80°C and 100°C, because it must be considered that in practice during a sanitization/disinfection cycle in some cases there will be a difference between the temperature of the items and the temperature of the gaseous atmosphere surrounding said items. Therefore, optionally and preferably the third heating system is configured to increase the temperature inside the exit antechamber to a third desired disinfection temperature, said third desired disinfection temperature preferably being of between 25°C and 100°C, and more preferably of between 40°C to 90°C, and most preferably of between 60°C and 90°C.

According to the above, optionally and preferably in the apparatus (system) of the first aspect of the invention the tubing system further comprises a third tubing circuit that has a third circuit inlet and a third circuit outlet, said third tubing circuit being connected to the exit antechamber via the third circuit inlet and the third circuit outlet, and moreover the device further comprises a third heating system that when operated is configured to increase a temperature inside the exit antechamber, the third heating system comprising a third heating element (or a plurality of third heating elements), a third auxiliary suction pump, and a third temperature sensor, the third heating element when operated being configured to heat inside the third tubing circuit, the third auxiliary suction pump when operated being configured to force any gas from inside the exit antechamber to successively exit said exit antechamber via said third circuit inlet, flow inside said third tubing circuit, and reenter the exit antechamber via the third circuit outlet.

Optionally and preferably, a method or an operation command related to operating the system (device), comprises operating or activating or deactivating the third heating system and one or more components of the third heating system, during any of the following and combinations thereof:
- before injecting (supplying) ozone to the disinfection chamber
- when injecting ozone to the disinfection chamber
- after injecting ozone to the disinfection chamber.

Preferably the third heating system is not being activated (i.e. is not switched on) when injecting ozone to the disinfection chamber.

For sanitizing some items with the apparatus, for example when applying a sanitization cycle, the third heating system heats for a total period of time that can also be called a third heating duration.

Preferably the electrical panel is configured for controlling independently the power supply to any of the different electrical or electronic or electromechanical components of the apparatus. Each of said components is connected, preferably via wire connections, to the power and control system. The motorized item transport system conveys the items when being activated by preferably the power and control system to which said transport system is preferably connected, the two systems being preferably configured so that the control system powers, and/or alternatingly activates and deactivates the motorized transport system so that the latter respectively conveys the items and stops conveying them.

Optionally and preferably the motorized item transport system comprises or is a conveyor system that extends through the disinfection chamber, the entrance door, the entrance antechamber, the external entrance door, the exit door, the exit antechamber and the external exit door.

Moreover, in the aforementioned case further optionally and preferably the conveyor system is a closed-loop conveyor system. In this case for example a conveyor belt of the conveyor system forms a closed loop that starts and ends on the same place, and in said place there can happen both the loading of the items on the conveyor system before the item's sanitization, and the unloading of the items from the system after the item's sanitization. This contributes to the system being compact.

When the transport system is a conveyor system, optionally and preferably the conveyor system extends over a loading and unloading area. The loading and unloading area is designated for loading and unloading thereat items on the conveyor system, and preferably said area is in front of any of the disinfection chamber, the entrance antechamber or the exit antechamber. This would allow the user to easily inspect the basic operation of the system while using it, without having to loose time moving around the system. It is important to point out that the loading and unloading of the items can optionally be done by a robot or other automated system.

Optionally and preferably the aforementioned conveyor system comprises a conveyor belt and/or a conveyor chain. According to the above, and in the case the motorized item transport system of the apparatus comprises or is a conveyor system, optionally and preferably the latter comprises a hanging conveyor system that preferably comprises a conveyor belt and a conveyor chain that is attached to and hanging from said conveyor belt, the conveyor chain having perforations adapted for hanging from the same said items, and/or for hanging hangers and/or containers and/or racks which are in turn adapted for placing on or hanging from the same the items. When the items can be hanged on the conveyor system (e.g. hanged from a chain in said system), then it can be said that the conveyor system is a hanging conveyor system. In that case, the part, e.g. the conveyor chain, from which the items are hanged is at a substantially elevated position with respect to the ground on which the apparatus resides, so that the hanging items (or hanging receptacles or hangers on which the items are placed) do not hit the floor as they are conveyed by the conveyor system. This configuration may also allow to the apparatuses' user having more space to move around as he/she works.

Moreover, optionally and preferably the apparatus' optional conveyor system comprises or is a closed-loop hanging conveyor system that extends over a loading and unloading area that is adapted for a user loading and unloading thereat items on the conveyor system. This can for example be an area over which passes the conveyor chain on which the items are hanged.

Optionally and preferably the conveyor system is adapted for hanging from the same the items to be sanitized and/or for hanging from the same hangers and/or racks and/or containers for loading thereon the items to be sanitized. Therefore, the items may not contact directly a movable chain or a belt of the conveyor system, but rather contact and be placed on, or be hanged from, a hanger or receptacle or a rack that hangs from said movable chain or belt of the conveyor system. This can offer the advantage of easily and quickly loading several items on the system, e.g. by first placing lots of items such as for example lots of pairs of shoes on an appropriately adapted rack and then quickly hanging said rack from the movable chain of the conveyor system. Likewise, in that case it would be easy to avoid touching directly the items when they have been sanitized, so that the chance of contaminating the items is reduced. When a user unloads the items from the conveyor system it may be sufficient to contact only a hanger or receptacle wherein said items are optionally located.

In the optional case that the system comprises a conveyor system as described above, then optionally and preferably the conveyor system comprises an electric motor adapted for moving the conveyor. Such motor optionally and preferably is configured to be controllable by the apparatuses' power and control system, and when the motor is activated/actuated by said control system, the motor in turn sets in motion the conveyor system and any items being thereon.

Based on the above, optionally and preferably the conveyor system comprises a rotor fitted with a respective electric motor that is controllable by the control and power system for rotating the rotor and moving the conveyor system and items thereon, wherein the rotor's shape preferably is polygonal, and more preferably is hexagonal. In a non-limiting example, the rotor resembles, or is, a circular or polygonal wheel that has a rim on which a conveyor belt of the conveyor system resides, and when the rotor rotates due to the action of a powered and activated motor, the conveyor belt moves thusly advancing/moving through the apparatus the items.

In the optional case that the system comprises a conveyor system as described above, then optionally and preferably the apparatus comprises a first lobby structure that is adjacent to the entrance antechamber and to the external entrance door and is adapted so that the items on the conveyor system can pass through the first lobby structure and along a first semicircular path therein, the first lobby structure preferably having a first semicylindrical shape. Likewise, when the system comprises the optional conveyor system, optionally and preferably the apparatus comprises a second lobby structure that is adjacent to the exit antechamber and the external exit door thereat and is adapted so that the items can pass through the second lobby structure along a second semicircular path therein, the second tunnel structure preferably having a second semicylindrical shape. The first and/or the second lobby structure offer multiple benefits: protect, at least partially, the external entrances of the apparatuses' antechambers from contamination such as from dust; prevent the user from accidently approaching said entrances of the antechambers wherein the concentration of ozone in the gases may be slightly elevated compared to the ambient environment especially in case of a malfunction of the system.

Effectively the first and the second lobby structures act as two tunnels via which the items respectively pass before entering the entrance antechamber and after exiting the exit antechamber. Likewise, said lobby's may be used for supporting or installing thereon parts of the mechanized transport system or of the optional conveyor system. In a particularly preferable optional case, the aforementioned optional rotor of the optional conveyor system is attached to a sealing of the optional first lobby structure, or to a sealing of the optional second lobby structure.

It is contemplated that optionally and preferably the conveyor system is a closed-loop conveyor system having a loop of a substantially discorectangle (obround) shape. In that case, the items when conveyed through the apparatus by the conveyor system, they move along a path having said discorectangle shape.

Likewise, it is contemplated the optional and preferable case wherein:
- the external entrance door and the (interior of the) entrance antechamber, the disinfection chamber, the (interior of the) exit antechamber and the external exit door are arranged in series (e.g. one after the other) along a first line segment of said discorectangle shape; and/or
- the aforementioned first lobby structure and the aforementioned first semicircular path are along a first semicircle of said discorectangle shape; and/or
- the aforementioned second tunnel structure and the aforementioned second semicircular path are along a second semicircle of said discorectangle shape.

Likewise, it is contemplated the optional case wherein the aforementioned optional loading and unloading area is arranged along a second line segment of said discorectangle shape. Overall, the aforementioned optional cases and features related to arranging the conveyor system's loop as a discorectangle, contribute to having an apparatus that is compact and easy to manufacture and install.

Optionally and preferably in the apparatus the gas extraction system comprises a first gas extractor that is connected to the entrance antechamber and configured for extracting from the latter gases. The optional first gas extractor preferably comprises a first extraction tube and a first electric fan therein. Said first extraction tube preferably projects from a ceiling of and above the entrance antechamber. Likewise, optionally and preferably in the apparatus the gas extraction system comprises a second gas extractor that is connected to the exit antechamber and is configured for extracting from the latter gases, the second gas extractor preferably comprising a second extraction tube and a second electric fan therein, the second extraction tube preferably projecting from a ceiling of and above the exit antechamber. When the extraction system comprises the aforementioned optional features, then the system is particularly safe and easy to use and maintain, since the first and second gas extractors offer improved extraction of ozone gas residues from the antechambers, and can in principle be operated and maintained independently with respect to each other. Moreover, these systems contribute to the system being compact and easy to install and maintain.

Likewise, it is contemplated the optional case wherein:
- the ozone destroy system and the gas extraction system are interconnected and configured so that when the apparatus is operated gases extracted with the gas extraction system are supplied to the ozone destroy system wherein any ozone with said gases is converted to a non-toxic gaseous product.

Likewise, it is contemplated the other optional case wherein:
- the gas extraction system comprises a second ozone destroy system that is configured to convert to a non-toxic gaseous product any gases extracted with the gas extraction system when the apparatus is operated.

In the aforementioned last two optional cases, it is achieved that the gases extracted by the extraction system can be passed via the ozone destroy system or via the second ozone destroy system, so that if there is ozone in said gases extracted by the gas extraction system, said ozone is destroyed so that it does not contaminate the atmosphere outside the apparatus. Said ozone may result from gas leaking from the disinfection chamber to the entrance and exit antechambers via the entrance door and the exit door when the latter two are closed or open. The second ozone destroy system may optionally comprise at least one catalyst filter (e.g. a filter comprising Carulite®) installed therein. For example, optionally each of the aforementioned optional first and second gas extractors may respectively comprise therein a first catalyst filter and a second catalyst filter configured to destroy (e.g. capture or convert to oxygen) ozone gas passing through them.

The apparatus's automatic doors when they are closed, aid to preventing/inhibiting ozone from escaping from the apparatus. In this context, optionally and preferably the entrance door, the exit door, the external entrance door and the external exit door are configured to close before or when the ozone generator generates ozone gas, or when the ozone destruction system is operated, when the apparatus is operated.

Likewise, it is contemplated the optional case wherein:
when or before the ozone destruction system is operated and/or when the gas extraction system is operated, the external entrance door and the external exit door are configured to be closed while the entrance door and the exit door are configured to be open. In the aforementioned last case it is easier to simultaneously destroy and remove ozone from both the system's antechambers and from the system's disinfection chamber.

Optionally and preferably, the entrance door and/or the exit door and/or the external entrance door and and/or the external exit door are configured to close non-hermetically. This is an optional feature that may come as a consequence of the optional case that said transport system comprises a conveyor that passes through said doors and prevents said doors from closing hermetically. The inventors have noticed that even when the doors do not close hermetically, the apparatus can work well and ozone concentration outside the apparatus can remain within safe levels.

Considering the aforementioned above, optionally and preferably the motorized item transport system comprises the aforementioned hanging conveyor system, and the entrance door and/or the exit door and/or the external entrance door and and/or the external exit door have portions which are configured to contact or surround the hanging conveyor system when any of the entrance door and/or the exit door and/or the external entrance door and and/or the external exit door close, and any of said doors excluding said portions closes hermetically. Therefore, when the apparatus comprises a conveyor belt as described above, any and preferably all of said doors when closing may optionally touch said conveyor belt and consequently, the doors may close hermetically across everywhere except where they contact said conveyor belt.

Alternatively to the above, optionally and preferably the entrance door and/or the exit door and/or the external entrance door and and/or the external exit door are configured to close hermetically. In a non-limiting optional example, the items are transported through the apparatus's antechambers and disinfection chamber by robots, wherein said robots are parts of the transportation system and are configured to not obstruct said doors when the latter close hermetically.

For allowing the user to better inspect/view the operations related to the use of the apparatus's chamber and/or antechambers, optionally and preferably:
- the disinfection chamber has a middle viewing transparent window that allows viewing within the disinfection chamber from outside; and/or
- the entrance antechamber has a first viewing transparent window that allows viewing within the entrance antechamber from outside; and/or
- the exit antechamber has a first viewing transparent window that allows viewing within the exit antechamber from outside,
Further optionally and preferably any of said middle, first or second window faces the aforementioned loading and unloading area.

For ensuring that the ozone gas supplied within the disinfection chamber is homogenously distributed therein for homogeneously sanitizing the items when the system is operated, optionally and preferably the apparatus comprises two ventilators located within the disinfection chamber. Preferably said two ventilators with respect to each other are located in opposite sides and more preferably on two opposite walls of the disinfection chamber, the ventilators being configured for forcing any gas within the disinfection chamber to move therein. Each ventilator preferably is an electric fan that is controlled/powered by the power and control system.

For protecting ozone generator and/or the ozone destroy system from accidental damages, optionally and preferably the apparatus comprises a compartment or a cabinet that is adjacent to and preferably behind the disinfection chamber and contains within it any or both of the ozone generator and an air or oxygen generator.

For having the apparatus' exhaust somewhere where it does not obstruct the user, optionally the end exhaust is on an exhaust tube that projects from above the disinfection chamber.

Likewise, for improving the apparatus' compactness, optionally the electrical panel is adjacent to and preferably behind the exit antechamber or the entrance antechamber.

For prolonging the apparatus' lifetime and improving its durability, optionally and preferably the interior of the disinfection chamber where the items are to be inserted is made of a material that preferably comprises any of an oxidation resistive material, a stainless steel, a chromium-nickel stainless steel, stainless steel 316, stainless steel doped with molybdenum and combinations therefrom. Generally, preferably any part of the apparatus that comes into contact with ozone, is made with one or more oxidation resistive materials such as the ones mentioned above.

The apparatus is advantageously used for sanitizing high volumes or numbers of items. Accordingly, preferably the apparatus is arranged to process such high volumes, and to be used in large scale operations, and for example in factories or large logistic centers. Therefore, optionally and preferably the interior of the disinfection chamber in which the items are to be inserted has a volume of between 100 liters (litres) and 10000 liters, and preferably of between 500 liters and 4000 liters, and most preferably of between 900 and 1500 liters.

Optionally and preferably the ozone generator comprises a precursor entrance via which a precursor gas enters the ozone generator when the latter generates ozone, the precursor gas preferably comprising ambient air or more preferably oxygen, and/or preferably wherein the ozone generator is a corona discharge ozone generator. Corona discharge ozone generators are reliable adding to the reliability of the device of the first aspect of the invention. Moreover, in the optional case wherein the precursor gas is ambient air, the ozone generator can directly draw (ambient) air and convert it, or part of it, to ozone.

Optionally the aforementioned precursor entrance communicates with a precursor opening at the main body, and via said precursor opening the precursor gas enters within the main body and within the ozone generator.

Optionally and preferably, said precursor gas comprises oxygen, and the apparatus further comprises an oxygen generator that is configured to generate oxygen and supply said oxygen to the aforementioned precursor entrance. Optionally and preferably said oxygen generator comprises a zeolite molecular sieve (ZMS) configured to absorb nitrogen molecules from the air and thusly produce high purity oxygen of a preferred concentration of between 90% and 97%.

Optionally and preferably in the apparatus the control and power system is configured to control the oxygen generator. For example, the control and power system (i.e. the power and control system) is preferably configured to activate and deactivate, e.g. to power on demand, the oxygen generator, and/or to adjust the oxygen generator's oxygen generation rate. Likewise, the oxygen generator is preferably configured to be activated by the control and power system, and for this purpose it may be connected via appropriate wiring with said control and power system.

It is contemplated that optionally and preferably the aforementioned oxygen generator is configured to generate oxygen gas at rate of between 0.1 litre of oxygen per minute (L/min) and 100 L/min, and preferably of between 1 L/min and 20 L/min, and more preferably of between 4 L/min and 12 L/min, and most preferably the oxygen generator is configured to generate oxygen gas at rate of 10 L/min, when the apparatus is operated. Said optional configurations offer the advantage of achieving the generation of enough oxygen for use in ozone generation so that the apparatus can function properly and within the operational parameters related to an efficient and fast disinfection.

Optionally and preferably the control and power system is configured to:
- supply electrical power of between 1 kW and 50 kW, and preferably of between 5 kW and 30 kW, and most preferably 25 kW; and/or
- consume between 5 kWh and 20 kWh of electrical energy when operated.

Also optionally the apparatus comprises a 63 A circuit breaker.

Optionally and preferably the apparatus of the first aspect of the invention comprises a cooling system configured to cool the ozone generator when the latter and the system are operated, the cooling system preferably being a water cooling system or an air cooling system, and wherein the control and power system and the cooling system are configured so that the former controls the latter. The cooling system ensures that the ozone generator is not overheated as a result of its operation. Preferably the cooling system is controlled by the control and power system e.g. the control and power system controls whether and/or when the cooling system is powered.

For accelerating and optimizing the disinfection it may be desirable to simultaneously treat the items and/or wearables to be disinfected with both ozone gas and ultraviolet radiation, especially UV-C radiation of a wavelength of between 100 nm and 280 nm. For this reason, optionally and preferably the apparatus of the first aspect of the invention comprises an ultraviolet (UV) radiation source located preferably on or within the disinfection chamber, wherein the control and power system is configured to control the operation of the UV radiation source, and the latter when operated is configured to generate and supply UV radiation within the disinfection chamber.

In the aforementioned case, optionally the UV radiation comprises any of UV-A, UV-B and UV-C, and combinations thereof, and preferably UV-C. The UV-A radiation has a wavelength of between 325 nm and 400 nm, and the UV-B radiation has a wavelength of between 280 nm and 315 nm.

In the aforementioned case, and for safety reasons, especially in the optional case wherein the disinfection chamber comprises a transparent window such as glass window, optionally and preferably the disinfection chamber comprises a UV filter that is installed on said transparent window and is configured to block and prevent UV-C and/or UV-B and/or UV-A radiation from passing through said transparent window.

The apparatus of the first aspect of the invention optionally and preferably comprises a suction pump, that is connected to the tubing system and is configured to be controllable by the power and control system when the apparatus is operated, the power and control system being configured to control the suction pump, and wherein the suction pump and the tubing system are configured to do any of the following and combinations thereof when the apparatus and suction pump are operated:
- force ozone gas and/or other gases, for example atmospheric air and/or oxygen, to move out of the disinfection chamber and through the exhaust inlet and through the exhaust outlet and towards and/or through the ozone destroy system;
- evacuate, e.g. evacuate the gaseous atmosphere out of, the disinfection chamber;
- force atmospheric air and/or oxygen gas to move into and/or through the tubing system or parts thereof, and/or into the disinfection chamber, preferably through the ozone outlet or through an air vent valve;
- force the non-toxic gaseous product to move out of the ozone destroy system and/or out of the system and/or through the end exhaust;
- force atmospheric air and/or oxygen gas to move into and/or through the tubing system and out of the ozone destroy system;
- force a precursor gas to enter the ozone generator.

Most preferably the suction pump is configured to move ozone gas from the disinfection chamber to the ozone destroy system. Therefore, preferably the suction pump is located and connected at the tubing system in between the ozone destruction means and the disinfection chamber. The suction pump may be an electric fan. Preferably the control unit (the power and control system) is connected via wire to the suction pump and configured to control the latter's powering and operation. When the suction pump operates while the ozone generator no longer generates and no longer supplies ozone to the disinfection chamber as is the preferred case when the disinfection is about to finish, the suction pump's action tends to create a vacuum within the disinfection chamber. To avoid creation of said vacuum, and to be able to clean said disinfection chamber from the ozone gas before the chamber can be opened for removing therefrom the sanitized items, optionally and preferably an air vent valve that preferably is a mechanical valve, is fixed on one of the walls of the disinfection chamber. Said air vent valve is configured to automatically open and allow atmospheric air from outside the main chamber to enter the latter when in said latter a vacuum tends to be created due to the action of the suction pump. Preferably the air vent valve is configured and/or graded to prevent the backflow through it of gas from the disinfection chamber to outside the latter.

Optionally and preferably, in said apparatus of the first aspect of the invention, the aforementioned sensing means comprise a lock sensor configured to sense whether the entrance door or the exit door or the external entrance door and or the external exit door is closed or not, and the safety condition comprises or is that the corresponding door is closed. The lock sensor preferably is an electronic and connected via wire to the control unit (power and control system), and the latter is configured to detect via the sensor whether the door is closed or open, and to prevent generation of ozone when the door is open so that a leak or an excessive leak of ozone via the open door is avoided. Most preferably, for each door there is a corresponding lock sensor.

Optionally and preferably the sensing means comprise an external ozone sensor that is located outside the disinfection chamber and preferably outside the entrance antechamber and the exit antechamber, and is configured to measure the concentration of ozone thereat, and the safety condition comprises or is that the said concentration of ozone measured by the external ozone sensor is less than a safe external concentration that preferably is of about 0.05 ppm (parts per million). Optionally the external ozone concentration is connected via wire to the power and control system, and both of them may be configured so that the latter controls the former and receives from it electric signals related to the measurements performed. If the ozone concentration measured by the external ozone sensor is of a value that is larger than 0.05 ppm, then said concentration may be toxic to the user, and the power and control systems preferably automatically shuts down the ozone generator or the entire system or apparatus. Obviously the external ozone sensor preferably is of the electronic type.

Optionally and preferably the sensing means comprise an internal ozone sensor that is located within the disinfection chamber or within the tubing system, and is configured to measure the concentration of ozone thereat, and preferably the safety condition is or comprises that the concentration of ozone measured by the internal ozone sensor is less than a maximum allowed internal concentration of preferably 10010 ppm or 4000 ppm or 3000 ppm or 2000 ppm. When the ozone concentration is larger than 10010 ppm, or is larger than one of the aforementioned possible maximum allowed concentration values, then the ozone may damage the items to be sanitized, and/or there may be an improper function of one of the device's components controlled by the control unit. Optionally the internal ozone concentration is connected via wire to the power and control system, and both of them may be configured so that the latter controls the former and receives from it electric signals related to the measurements performed. Obviously the internal ozone sensor preferably is of the electronic type.

According to the above, optionally and preferably the sensing means are electronic and connected via wire to the power and control system, and the sensing signal is an electrical signal transmitted via said wire, and the power and control system and the sensing means are configured so that the power and control system can detect and/or process said electric signal and/or can control the sensing means.

Optionally and preferably in the apparatus the ozone destroy system comprises a catalytic ozone destructor comprising a catalyst wherein preferably the catalyst comprises manganese oxide and/or copper oxide. In a preferred but not limiting example, the catalyst comprises or is a CARULITE® 200 catalyst.

Moreover, optionally and preferably the ozone destroy system comprises a deodorization system, such as for example a deodorization filter, that is configured to deodorize or aromatize the non-toxic gaseous product. In a non-limiting contemplated example, the aforementioned deodorization filter is an active carbon or sepiolite filter, and the ozone destroy system comprises the deodorization filter connected in series with a catalytic ozone destructor, and the system is configured so that the gasses processed by the ozone destroy system first are processed by the catalyst filter and subsequently are processed by the deodorization filter. A deodorization filter is preferably included when the system is destined to be used within a cloth store or storage facility wherein the release by the system of unpleasant smells is to be avoided.

Optionally and preferably the apparatus according to the first aspect of the invention comprises a ventilation system configured to homogenize the ozone gas concentration within the disinfection chamber when the system and the ozone generator are operated, wherein preferably the ventilation system comprises two ventilators located within the disinfection chamber, and preferably the two ventilators with respect to each other are located in opposite sides and more preferably on two opposite walls of the disinfection chamber, the ventilators being configured for forcing any gas within the disinfection chamber to move therein, each ventilator preferably being an electric fan, the ventilation system and the power and control system being configured so that the latter controls the former. Essentially the optional ventilation system is destined to force the ozone gas to disperse through the volume of the disinfection chamber and flow therein so the surfaces of the items being disinfected are well treated homogenously and continuously.

When the apparatus comprises the aforementioned ventilation system, then preferably said ventilation system comprises an electric fan located in the disinfection chamber or in or connected to the tubing system, and is configured to increase the travel speed or flow of any gas, such as ozone, that enters via the ozone outlet or is within the disinfection chamber. Preferably the ventilation system comprises two or more electric fans. Said optional ventilation system and the power and control system are preferably configured so that the latter controls the former.

Optionally and preferably in an apparatus according to the first aspect of the invention the user command means comprise a power button or knob and the like configured for switching on or off the power supply. In this optional case, the user can switch on and switch off the power supply and the system by handling said power button or knob.

Moreover, optionally and preferably the user command means comprise an emergency button configured for stopping all operation of the apparatus without interrupting the power supply of the same. In this optional case, the user can stop all operation of the apparatus without interrupting the power supply of the same by handling said emergency button.

Moreover, optionally and preferably the user command means comprise a reset button configured for restarting operation of the apparatus or of the power and control system or of the computer therein in case of a failing. Preferably for security reasons said reset button has to be used by the user every time the apparatus is switched on.

Optionally and preferably the user command means comprise an electronic touchscreen. Likewise, optionally and preferably the user control means are configured to display to the user several operation command options and sense a touching or selection of any of said operation command options by the user, and the computer is configured to convert said touching or selection of any of said operation command options to a corresponding operation command that is pre-stored and/or programmed in the computer or in a non-transitory computer readable medium connected to and accessed by the computer or to a remote server that in turn is connected, e.g. via the internet or a computer network, to the computer. Non limiting examples of said non-transitory computer readable medium are a magnetic or an optical disc, a hard drive, a flash memory card (e.g. SD card), a CD, a USB stick and the like. The computer may be PLC. In a non-limiting example related to preferred embodiment of the device according to the first aspect of the invention, the electronic operation command options are displayed via a touchscreen, and comprise icons related to activating or deactivating, such as powering on or off, any of the system's components, such as the ozone generator or the ozone destroy system or the item transport system or the gas extraction system or any of the apparatus' automatic doors. More preferably an electronic command option relates to the coordinated activation and deactivation and operational control by the control unit (power and control system) of the device's components for thusly executing with the device a sanitization (disinfection) cycle. The sanitization cycle preferably includes checking whether the safety condition is met and executing, preferably in a consecutive manner, the following: opening all the doors; setting in motion the transportation system until detecting that an item loaded on the transportation system or a specific part of the transportation system is inside the disinfection chamber; closing all automatic doors; activating the gas extraction system and the ozone generator and the ventilation system for generating and passing and dispersing ozone to/in the disinfection chamber for a desired period of time while preventing said ozone from uncontrollably leaking outside the apparatus; deactivating the ozone generator and activating the ozone destroy system and the suction pump for removing ozone from the disinfection chamber and filling said chamber with atmospheric air; opening the entrance and exit door and continue operating the ozone destruction system and the gas extraction system until the ozone has been completely or nearly completely removed from the disinfection chamber and any of the two antechambers; stopping the ozone destruction system and optionally stopping the gas extraction system; opening the external entrance door and the external exit door; setting motion in the transportation system so that the items exit the disinfection chamber and the antechambers; and optionally indicating to the user that the sanitization cycle has been completed. The apparatus preferably comprises electric locking mechanisms that are connected to and/or are controlled by the power and control system, and said locking mechanisms are configured to lock the automatic doors while the ozone generator is operated. Therefore, an operation command option may include keeping activating said locking mechanism for a specific period of time or while certain conditions exist. Each operation command option preferably is related to a corresponding command that is pre-stored and/or programed to the computer or PLC. The optional touchscreen and the computer or PLC connected to said touchscreen can optionally be configured so that via the touchscreen the user may program the sanitization (disinfection) cycles, or may activate and/or control individual components of the system.

Optionally and preferably the tubing system comprises an air vent with an air vent valve therein configured to let air enter the tubing system and/or the disinfection chamber when the air vent valve is open, wherein said air vent valve is configured to be controllable by the power and control system or is mechanical. The air vent valve can be considered to be similar to the air vent valve described further above.

Optionally and preferably the entrance antechamber comprises an air vent with a second air vent valve therein configured to let air from outside the apparatus to enter the entrance antechamber when the second air vent valve is open, wherein said second air vent valve is configured to be controllable by the power and control system or is mechanical.

Optionally and preferably the exit antechamber comprises an air vent with a third air vent valve therein configured to let air from outside the apparatus to enter the exit antechamber when the third air vent valve is open, wherein said third air vent valve is configured to be controllable by the power and control unit or is mechanical.

The aforementioned optional air vent valves can be similar between them, and each serve for avoiding creating vacuum within the (ante)chambers when any of the fans or pumps optionally connected thereat removes gases from said (ante)chambers. Likewise said air vent valves serve for reducing the concentration of any ozone that may be within said (ante)chambers, as part of the sanitization cycle that is implemented with the apparatus.

According to some of the above, optionally and preferably the apparatus of the first aspect of the invention comprises a position sensor that is configured to sense the position of a part of the motorized transport system and/or of an item loaded on the transport system, and said position sensor is preferably configured to sense whether said part or item is located within the disinfection chamber, and to generate a respective position sensing signal when said part or item is within the disinfection chamber. Preferably the position sensor is connected to the power and control system and is controllable by the latter, the power and control system being configured to receive said position sensing signal. A non-limiting example of a position sensor is a photocell that is configured to sense whether an item approaches said photocell.

Optionally and preferably in an apparatus according to the first aspect of the invention, one of said operation commands (instructions) that is pre-stored and/or programmed in the computer or in a non-transitory computer readable medium connected thereat, comprises any of the following or combinations thereof or preferably all of:
- activate and/or set in motion the mechanized transport system until the position signal is generated;
- close the entrance door and the exit door and the external entrance door and the external exit door;
- activate (use) the second heating system to increase the temperature inside the entrance antechamberto a desired second disinfection temperature;
- deactivate the second heating system;
- activate (use) the third heating system to increase the temperature inside the exit antechamber to a desired third disinfection temperature.
- deactivate th third heating system.
- activate (use) the heating system to increase the temperature inside the disinfection chamber to a desired disinfection temperature.
- deactivate the heating system.
- activate the oxygen generator to generate oxygen;
- activate the gas extraction system to extract gases from the entrance antechamber and the exit antechamber;
- activate the ozone generator to generate ozone gas, preferably for a total duration of between 1 second and 20 minutes, or for an ozone generation duration of between 1 second and 10 minutes (min), or for a disinfection duration of between 1 minute (min) and 20 min, and preferably of between 2 min and 10 min, while preferably measuring (detecting) with the internal ozone sensor an ozone concentration of between 1 ppm and 10000 ppm;
- activate/operate the cooling system and using the same to cool the ozone generator while generating with the ozone generator ozone gas;
- activate the ventilation system to homogenize the ozone gas concentration within the disinfection chamber, preferably while generating with the ozone generator ozone gas;
- deactivate or disconnect or shut down or stop operating the ozone generator to stop generating ozone, and optionally, deactivate or disconnect or shut down or stop operating the oxygen generator and/or the cooling system and/or the ventilation system;
- activate/operate operating the suction pump to remove ozone from the disinfection chamber, and open the air vent valve when the latter is controllable by the power and control system;
- activate/operate the ozone destroy system thusly destroying ozone gas;
- open the exit door and the entrance door;
- deactivate/stop the ozone destruction system and/or the suction pump, preferably when the internal ozone sensor measures an ozone concentration of less than 0.05 ppm and/or when a safety period of time since stopping the ozone generator has passed;
- open the external entrance door and the external exit door, and preferably deactivate the gas extraction system;
- activate and/or set in in motion the mechanized transport system to move the items outside the disinfection chamber and preferably outside the entrance and the exit antechambers;
- preferably, indicate to the user via the touchscreen that the operation command has been executed.

Optionally and preferably the items to be sanitized with the apparatus are clothes and/or shoes and/or linens. Likewise, optionally the items are any of clothes, apparel, clothing accessories, trousers, shoes, hair caps, personal protective equipment (PPE) such as protective clothing, helmets, goggles, face masks or other garments or equipment designed to protect the wearer's body from injury or infection, and combinations thereof.

It is contemplated the optional and preferable case wherein the apparatus according the first aspect of the invention further that comprises any or both of:
- a humidity sensor located within the disinfection chamber or attached to or within the tubing system and configured to measure thereat a humidity concentration and generate and transmit a corresponding humidity signal;
- a humidity control system configured to control the humidity within the disinfection chamber by introducing humidity, or removing humidity from within the disinfection chamber;
wherein preferably the power and control system is configured to control the humidity sensor and receive said humidity signal, and/or is configured to control the operation of the humidity control system. The humidity control system is configured to be controlled by the power and control system. The power and control system is preferably configured to operate the humidity control system to cause that the humidity concentration measured by the humidity sensor acquires a preferred humidity concentration value. The preferred humidity concentration value preferably is of between 8% and 80%, and more preferably of between 40% and 70%, and most preferably of between 60% and 70%. Having said preferred humidity concentration values may result to improving the efficacy and efficiency of the sanitization achieved with the system because water is known to affect the physicochemical effects of ozone treatment on surfaces. Likewise, the humidity value can be adjusted considering the specific items to be sanitized and the materials therein. The humidity control system may comprise a humidifier.

Moreover, it is contemplated the optional and preferred case wherein, the operation command and/or the related operation command option is related to the material or type of the items or wearables to be disinfected, or the quantity or volume of the items to be disinfected. For example, an operation command may include specifying a type of item or wearable or characteristic therein such as a material of the wearable, and/or adjusting a recipe (sanitization cycle) parameter according to said specifying item or wearable or characteristic, said recipe parameter being any of the following and combination thereof:
- disinfection/sanitization time;
- ozone generation rate;
- preferred Humidity value or range;
- maximum allowed internal concentration of ozone (as measured by an internal ozone concentration sensor);
- ozone concentration value or value range as measured by an internal ozone sensor;
- ozone generation duration;
- first ozone destruction time;
- second ozone destruction time;
- safety period of time;
- desired disinfection (sanitization) temperature;
- desired second disinfection temperature;
- desired third disinfection temperature;
- Heating duration;
- A second heating duration;
- A third heating duration.

For example the operation command may comprise accessing a computer file according to which an initial condition is correlated with at least one value or value range of any of the recipe parameters described above or combinations thereof, said condition being any of a specific material to be disinfected, or an item to be disinfected, or a volume or quantity of items to be disinfected, or a specific level of disinfection required, or a specific protocol of disinfection required, and wherein the operation command may further comprise controlling the system and components thereof as required, to realize, as part of implementing a disinfection cycle, the recipe parameter value or value range that correlates to the initial condition.

The invention in its second aspect is (concerns) a method for sanitizing and/or disinfecting an item or items with an apparatus, wherein said apparatus comprises a conveyor system, a disinfection chamber that has an entrance door and an exit door, an entrance antechamber that is adjacent to said disinfection chamber and to the entrance door and has an external entrance door, an exit antechamber that is adjacent to the disinfection chamber and to the exit door and has an external exit door, an ozone generator, an ozone destroy system, a power and control unit (power and control system), and a gas extraction system that preferably comprises a first gas extractor connected to the entrance antechamber and a second gas extractor connected to the exit antechamber, the apparatus preferably being according to the first aspect of the invention, the method comprising:
- placing the items on the conveyor system;
- setting in motion the conveyor system for moving and inserting the item (or items) to be disinfected to within the disinfection chamber;
- stopping the motion of the conveyor system;
- closing the entrance and the exit doors and the external entrance door the external exit door;
- activating the gas extraction system thusly extracting gases from the entrance and the exit antechambers;
- activating the ozone gas generator thusly generating ozone gas, and supplying said ozone gas to the disinfection chamber for disinfecting the items;
- deactivating the ozone gas generator;
- activating the ozone destruction system for removing ozone gas from the disinfection chamber and converting said ozone gas to a non-toxic gaseous product;
- opening the entrance and exit doors;
- operating the gas extraction system for preferably a safety period of time before preferably deactivating the gas extraction system;
- deactivating the ozone destruction system;
- opening the external entrance door and the external exit door;
- setting in motion the conveyor system for moving and removing the sanitized item (or items) from within the disinfection chamber;
- removing the items from the conveyor system.

Optionally and preferably the method according to the second aspect of the invention further comprises:
- activating the heating system, or activating any elements of said heating system, such as activating for example the auxiliary suction pump and the heating element, for increasing the temperature inside the disinfection chamber to, preferably, a desired disinfection temperature.
- deactivating the heating system, or activating any elements of said heating system, such as activating for example the auxiliary suction pump and the heating element.

Optionally and preferably the method according to the second aspect of the invention further comprises:
- activating the second heating system, or activating any elements of said second heating system, such as activating for example the second auxiliary suction pump and the second heating element, for increasing the temperature inside the entrance antechamber to, preferably, a desired second disinfection temperature.
- deactivating the second heating system, or activating any elements of said second heating system, such as activating for example the second auxiliary suction pump and the second heating element.

Optionally and preferably the method according to the second aspect of the invention further comprises:
- activating the third heating system, or activating any elements of said third heating system, such as activating for example the third auxiliary suction pump and the third heating element, for increasing the temperature inside the exit antechamber to, preferably, a desired third disinfection temperature.
- deactivating the third heating system, or activating any elements of said third heating system, such as activating for example the third auxiliary suction pump and the third heating element.

The apparatus used with the method of the second aspect preferably is according to the first aspect of the invention. Therefore, any optional or essential features of the apparatus described further above in relation to the first aspect of the invention, can also be corresponding features of the apparatus used with the method of the second aspect of the invention. Likewise, any actions related to the operation or functionality of the apparatus and its elements, can be actions or steps of the method of the second aspect of the invention.

Herein the power and control system is also being referred to as control unit or as power and control unit.

Optionally and preferably the items to be sanitized with the method of the second aspect of the invention are any of clothes, apparel, clothing accessories, towels, home textiles, linens, towels, trousers, shoes, jackets, coats, dresses, hair caps, wearables, personal protective equipment (PPE) such as protective clothing, helmets, goggles, face masks or other garments or equipment designed to protect the wearer's body from injury or infection, and combinations thereof.

In the method of the second aspect of the invention, optionally and preferably placing the items to be sanitized comprises placing the items in or on a receptacle or hanger or rack that in turn is placed or hanged from the conveyor system. Likewise, optionally and preferably removing the items comprises avoiding touching the items, and/or comprises packeting the items within a plastic foil or bag, and/or comprises wrapping the items in said plastic foil or bag, and/or comprises placing a sign on the items or on a packet containing them wherein said sign indicates that the items have been sanitized. Likewise, optionally and preferably removing the items may optionally comprise avoiding touching the receptacle or hanger. When the items are loaded, or unloaded or (em)packeted or signed as described above, it is more difficult to accidently contaminate them while subsequently handling them.

Optionally and preferably in the method of the second aspect of the invention, after removing the disinfected items from the conveyor system optionally the method further comprises any of the following and combinations therefrom:
- placing the items or a receptacle or hanger having them within a package that for example is a second parcel or a sealable plastic bag or a plastic wrap, while preferably avoiding touching the items or a receptacle containing them;
- placing a sign on said second parcel indicating that the items are disinfected or sanitized.

For promoting that a user of the apparatus does not accidently contaminate the items, and/or that the user follows a safety protocol, optionally and preferably said method of the second aspect of the invention further comprises the user wearing personal protective equipment while applying the method or parts thereof using the apparatus.

For ensuring a rapid sanitization cycle and high productivity while avoiding using excessive ozone concentrations that would potentially damage the items, optionally and preferably in the method of the second aspect of the invention, generating ozone gas using the ozone generator comprises generating ozone gas at a rate of between 1 grams of ozone per hour to 100 grams of ozone per hour, and preferably of between 50 grams of ozone per hour to 60 grams of ozone per hour and supplying said ozone gas to the disinfection chamber. Likewise, optionally and preferably generating ozone gas using the ozone generator and supplying said ozone gas to the disinfection chamber is optionally performed for an ozone generation duration of between 1 second and 10 minutes (min) or preferably of between 30 seconds and 5 min, or for a disinfection duration of between 1 second (s) and 20 min, and preferably of between 30 seconds and 10 min.

Optionally and preferably the method of the second aspect of the invention further comprises supplying said ozone gas to the disinfection chamber at an ozone concentration of between 1 ppm and 10000 ppm as measured by using an ozone sensor thereat, e.g. using an internal ozone sensor located at the disinfection chamber or at a tubing system that connects the ozone generator and the disinfection chamber and via which the ozone gas is being supplied. The indicated above ozone concentration values relate to an optimum treatment in terms of achieving quick and efficient sanitization cycles while avoiding damaging the items.

Optionally and preferably the method of the second aspect of the invention comprises stopping passing said ozone gas to the disinfection chamber. Likewise, optionally and preferably the method comprises converting said ozone gas to a non-toxic gaseous product, using the ozone destruction system, until the ozone concentration in gas within the disinfection chamber as measured by the aforementioned internal ozone sensor is less than 0.05 ppm, or until a safety period of time has passed.

Optionally and preferably the method of the second aspect of the invention further comprises: while supplying said ozone gas to the disinfection chamber controlling the humidity within the disinfection chamber by introducing humidity within the disinfection chamber, or removing humidity from within the disinfection chamber. The humidity is a parameter that when controlled, the speed and efficacy or efficiency of the sanitization method may be further optimized. Likewise, optionally and preferably controlling the humidity comprises causing that the humidity concentration within the chamber has or acquires a preferred humidity concentration value which preferably is of between 8% and 80%, and more preferably of between 40% and 70%, and most preferably of between 60% and 70%.

Optionally and preferably in the method of the second aspect of the invention the apparatus is within any of a healthcare facility, a hospital, a medical center, a public service building, a school, a factory, a store, a laundry shop, a goods storage facility, a food production or processing facility or open field, a logistics center, a facility wherein cloths or apparel are made or processed or stored, and the items are any of clothes, apparel, trousers, shoes, hair caps, personal protective equipment (PPE) such as protective clothing, helmets, goggles, face masks or other garments or equipment designed to protect a wearer's body from injury or infection, and combinations thereof.

The invention in its third aspect is a software or a non-transitory computer readable medium that contains instructions and/or commands and/or command options related to operating the apparatus of the first aspect of the invention, e.g. implementing a sanitization cycle, and/or related to implementing the method of the second aspect of the inventions or steps and processed therein.

### Brief Description of the Drawings

The previous and other advantages and features will be more fully understood from the following detailed description of embodiments, with reference to the attached figures, which must be considered in an illustrative and non-limiting manner, in which:
FIG 1 is a schematic diagram of a preferred embodiment of an apparatus according to the first aspect of the invention.
FIG 2 is a perspective view diagram of the preferred embodiment of the apparatus according to FIG 1.
FIG 3 is a side view (view from the side) of the apparatus shown in FIG 2.
FIG 4 is a top view (view from the top) of the apparatus shown in FIG 2.
FIG 5 is a back view (view from the top) of the apparatus shown in FIG 2.
FIG 6 is a top view (view from the top) of the apparatus shown in FIG 2 but without part of the top ceiling of said apparatus so that the shape and an aspect of a conveyor system of the apparatus is shown.
FIG 7. is a perspective view of a part of the transportation system, which in this case comprises a conveyor system, of an apparatus of an embodiment that is very similar to the one shown in FIG 2, without part of the top ceiling of said apparatus.
FIG 8 is a more detailed drawing of the perspective view drawn of FIG 7.
FIG 9 is a perspective view diagram of an apparatus of an embodiment that is very similar to the one shown in in FIG 2, without part of the top ceiling of said apparatus.
FIG 10 shows a flow for the basic parts of the method related to operating the apparatus shown in FIG 2.
FIG 11 is a flow of the basic states in a preferred embodiment of the method or a sanitization cycle therein.
FIG 12 is a flow of the steps (actions) for transitioning from the first basic state to the second basic state in a preferred embodiment of the method or a sanitization cycle therein.
FIG 13 is a flow of the steps (actions) for transitioning from the second basic state to the third basic state in a preferred embodiment of the method or a sanitization cycle therein, and also shows the flow of sub-steps (sub-actions) within said steps.
FIG 14 is a flow of the steps (actions) for transitioning from the third basic state to the first basic state in a preferred embodiment of the method or a sanitization cycle therein, and also shows the flow of sub-steps (sub-actions) within said steps.
FIG 15 is a flow for transitioning from a fourth basic state to any of the first, the second or the third basic state and eventually to the Finish during a preferred embodiment of the method or a sanitization cycle therein.
FIG 16 is a schematic diagram indicating the configuration in a preferred embodiment of an apparatus according to the first aspect of the invention, wherein the apparatus is at the first state.
FIG 17 is a schematic diagram indicating the configuration in a preferred embodiment of an apparatus according to the first aspect of the invention, wherein the apparatus is at the second state.
FIG 18. is a schematic diagram indicating the configuration in a preferred embodiment of an apparatus according to the first aspect of the invention, wherein the apparatus is at the third state.
FIG 19a is a front view of a preferred embodiment, with some dimensions measured in millimeters (mm) being indicated therein.
FIG 19b is a top view of the preferred embodiment shown 19a, with some dimensions measured in mm being indicated therein.
FIG 19c is a perspective view of the preferred embodiment shown in FIG 19a.
FIG 20 is a schematic diagram that shows only a part of a preferred embodiment of an apparatus according to the first aspect of the invention.
FIG 21. is a schematic diagram that shows only a part of a preferred embodiment of an apparatus according to the first aspect of the invention.
FIG 22. is a schematic diagram that shows only a part of a preferred embodiment of an apparatus according to the first aspect of the invention.

### Detailed Description of Preferred Embodiments

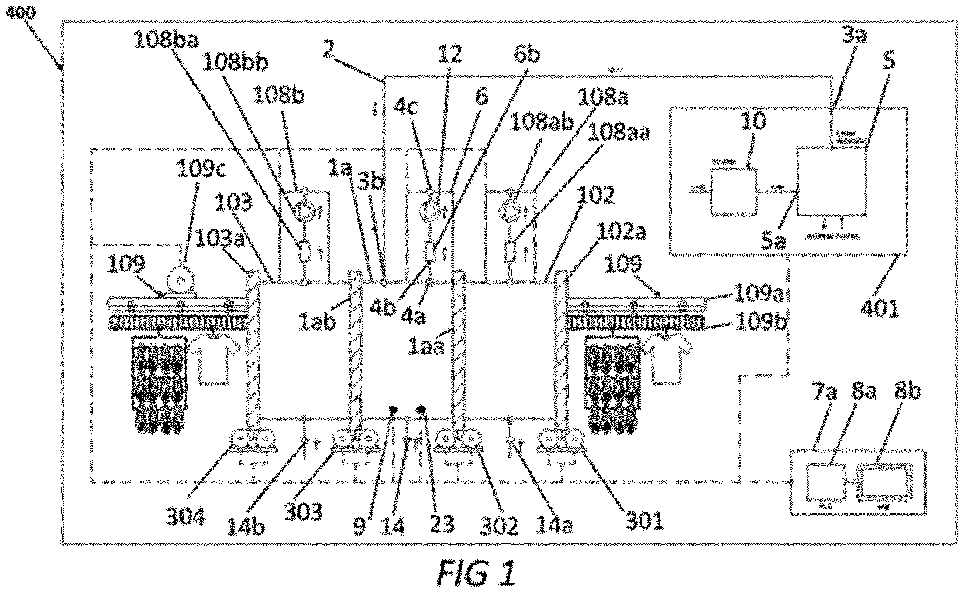

FIG 1 describes schematically the configuration of a preferred embodiment of the apparatus 400 according to the first aspect of the invention. As seen in FIG 1 the apparatus comprises:
- a disinfection chamber 1a which has an entrance door 1aa and an exit door 1ab and is configured so that the items to be sanitized enter within the disinfection chamber 1a, via the entrance door 1aa, and exit from the disinfection chamber 1a via the exit door 1ab, the entrance door 1aa and the exit door 1ab being automatic;
- a tubing system 2 that has an ozone inlet 3a, an ozone outlet 3b, an exhaust inlet 4a, an exhaust outlet 4b and an end exhaust 4c, wherein the tubing system 2 is connected to the disinfection chamber 1a via the ozone outlet 3b and the exhaust inlet 4a, and is configured to, during an operation of the apparatus by a user, supply to the disinfection chamber 1a ozone gas via the ozone outlet 3b, and to receive from the disinfection chamber 1a ozone gas via the exhaust inlet 4a;
- an entrance antechamber 102 that has an external entrance door 102a and is adjacent to the disinfection chamber 1a and to the entrance door 1aa thereat, and is configured and arranged so that the items before entering the disinfection chamber 1a pass via the external entrance door 102a and through the entrance antechamber 102, the external entrance door 102a being automatic;
- an exit antechamber 103 that has an external exit door 103a and is adjacent to the disinfection chamber 1a and to the exit door 1ab thereat, and is configured and arranged so that the items upon exiting the disinfection chamber 1a pass through the exit antechamber 103 and exit therefrom via the external exit door 103a, the external exit door 103a being automatic;
- a motorized item transport system that is configured to convey the items so that, when the apparatus is operated, the items successively pass through the external entrance door 102a and the entrance antechamber 102, enter via the entrance door 1aa and pass through the disinfection chamber 1a and exit from the latter via the exit door 1ab, and pass through the exit antechamber 103 exiting therefrom via the external exit door 103a;
- an ozone generator 5 connected to the tubing system 2 and configured to generate ozone gas and supply via the ozone inlet 3a said ozone gas to the tubing system 2 when the apparatus is operated, the ozone generator preferably being configured to generate ozone at a rate of between 1 grams of ozone per hour to 100 grams of ozone per hour, and preferably of between 50 grams of ozone per hour to 60 grams of ozone per hour and supplying said ozone gas to the disinfection chamber;
- an ozone destroy system 6 connected to the tubing system 2 and is configured to receive from the latter via the exhaust outlet 4b ozone gas that has been received by the tubing system 2 via the exhaust inlet 4a, and to convert said ozone gas to a non-toxic gaseous product, and to supply said non-toxic gaseous product to the end exhaust 4c;
- a gas extraction system that is configured to extract gases from within the exit antechamber 103 and from the entrance antechamber 102 when the apparatus is operated;
- sensing means 9 configured for sensing at least one operational parameter and for generating and transmit a corresponding sensing signal when the apparatus is operated;
- a control and power system 7a for controlling operating the apparatus, the control and power system 7a comprising an electrical panel for controlling powering the apparatus, a computer 8a and user command means 8b configured for receiving operation commands from a user, the computer preferably being a programmable logic controller, the user command means 8b preferably comprising a touchscreen, and the control and power system 7a is configured to control the operation of the apparatus according to the operation commands, and is configured to receive the sensing signal and inhibit the operation of the apparatus when the sensing signal does not correspond to a safety condition.

The exit door 1ab comprises an exit door actuation mechanism 303, the entrance door 1aa comprises an entrance door actuation mechanism 302, the external exit door 103a comprises an external exit door actuation mechanism 304, and the external entrance door 102 a comprises an external entrance door actuation mechanism 301, and each of said actuation mechanisms is connected to and configured to be controllable by the power and control system 7a. Said actuation mechanisms are configured to open and close the door when activated accordingly.

The dashed lines in FIG 1 indicate wired connections between the power and control system 7a (i.e. the control and power system) and the various electrical or electromechanical or electronic components of the apparatus that are controllable by said power and control system 7a.

As shown in FIG. 1 the ozone generator 5 comprises a precursor entrance 5a via which a precursor gas enters the ozone generator 5 when the latter generates ozone; in this case said precursor gas comprises oxygen and the apparatus 400 further comprises an oxygen generator 10 that is configured to generate oxygen and supply said oxygen to the precursor entrance 5a. The ozone generator and the oxygen generator form together an ozone generation system 401 that is connected to and controllable by the power and control system 7a.

In the apparatus 400 shown in FIG 1, the motorized item transportation system is a conveyor system 109 that comprises a rotor (the rotor in this case is a rotatable wheel) 109c fitted with a respective electric motor that is controllable by the control and power system 7a for rotating the rotor and moving (setting in motion) the conveyor system and items thereon. Moreover, the conveyor system 109 is a hanging conveyor system that comprises a conveyor belt 109a and a conveyor chain 109b that is attached to and hanging from said conveyor belt 109a, the conveyor chain 109b having perforations adapted for hanging from the same said items, and for hanging hangers and/or containers and/or racks which are in turn adapted for placing on or hanging from the same the items. The rotor in this case is (has the shape of) a rotatable wheel and the conveyor belt in this case contacts said wheel and is moved by the wheel when the latter is rotated by the action of the motor when said motor is activated. FIG 1 shows a t-shirts and pairs of shoes hanging respectively from hangers and racks that are hanging from the conveyor chain 109b.

In the case of the apparatus of FIG 1 the sensing means 9 comprise an internal ozone sensor that is located within the disinfection chamber 1a.

In FIG 1 it is indicated that the apparatus 400 comprises a ventilation system 23 configured to homogenize the ozone gas concentration within the disinfection chamber when the system and the ozone generator are operated, wherein in this case the ventilation system comprises two ventilators located within the disinfection chamber, and the two ventilators with respect to each other are located in on two opposite walls of the disinfection chamber 1a, each ventilator being an electric fan.

As indicated in FIG 1 the apparatus and more specifically in this case the ozone destruction system 6 comprises a suction pump 12, that is connected to the tubing system 2 and is configured to be controllable by the power and control system 7a when the apparatus is operated, and the suction pump 12 and the tubing system 2 are configured to force gases to move out of the disinfection chamber 1a and through the exhaust inlet 4a and through the exhaust outlet 4b and towards the end exhaust 4c. The ozone destruction system shown in FIG 1 also comprises a catalytic ozone destructor 6b comprising a catalyst.

As shown in FIG 1 the gas extraction system in the particular embodiment comprises:
- a first gas extractor 108a connected to the entrance antechamber 102 and configured for extracting from the latter gases, the first gas extractor comprising a first extraction tube 108aa and a first electric fan 108ab therein.
- a second gas extractor 108b connected to the exit antechamber 103 and configured for the extracting from the latter gases, the second gas extractor comprising a second extraction tube 108ba and a second electric fan 108bb therein.

Moreover, as shown in FIG 1 in the apparatus 400:
- the tubing system 2 comprises an air vent with an air vent valve 14 therein configured to let air enter the disinfection chamber 1a when the air vent valve is open, wherein said air vent valve in this case is mechanical;
- the entrance antechamber 102 comprises an air vent with a second air vent valve 14a therein configured to let air from outside the apparatus to enter the entrance antechamber 102 when the second air vent valve is open, wherein said second air vent valve 14a in this case is mechanical;
- the exit antechamber 103 comprises an air vent with a third air vent valve 14b therein configured to let air from outside the apparatus to enter the exit antechamber 103 when the third air vent valve is open, wherein said third air vent valve 14b in this case is mechanical.

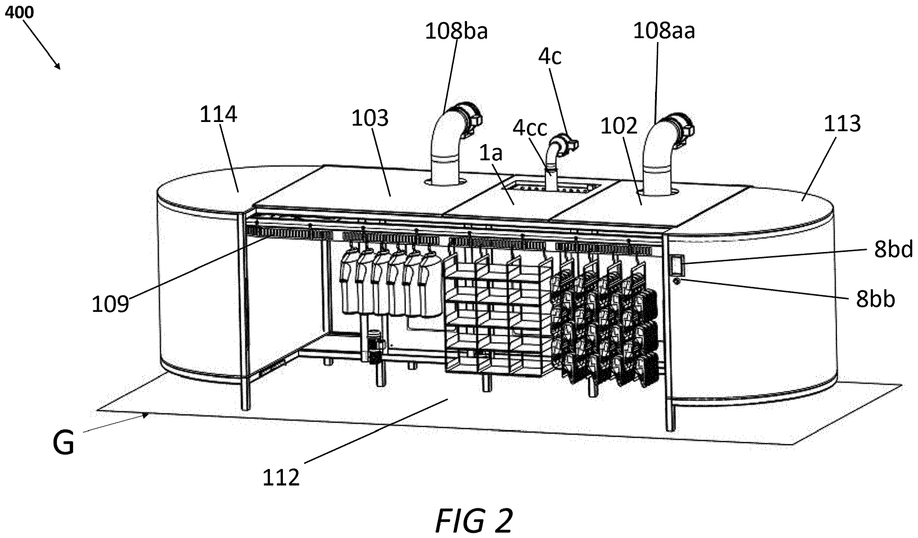

Fig. 2 shows that the apparatus according to FIG 1 further comprises
- a first lobby structure 113 that is adjacent to the entrance antechamber 102 and the external entrance door and is adapted so that the items on the conveyor system can pass through the first lobby structure 113 and along a first semicircular path, the second lobby structure having a first semicylindrical shape.
- a second lobby structure 114 that is adjacent to the exit antechamber 103 and the external exit door thereat and is adapted so that the items can pass through the second lobby structure 114 along a second semicircular path, the second tunnel structure having a second semicylindrical shape.

Moreover, in Fig. 2 it is shown that:
- the first extraction tube 108aa projects from a ceiling of and above the entrance antechamber 102;
- the second extraction tube 108ba projects from a ceiling of and above the exit antechamber 103;
- the end exhaust 4c is on an exhaust tube 4cc that projects from above the disinfection chamber 1a;
- the user command means comprise a touchscreen 8bd, and they also comprise an emergency button 8bb.

As shown in FIG 2 the conveyor system 109 extends over a loading and unloading area 112 on the level/ground G on which the apparatus resides. The loading and unloading area 112 is designated for loading and unloading thereat items on the conveyor system, and said area 112 is in this case in front of the disinfection chamber 1a, the entrance antechamber 102 and the exit antechamber 103.

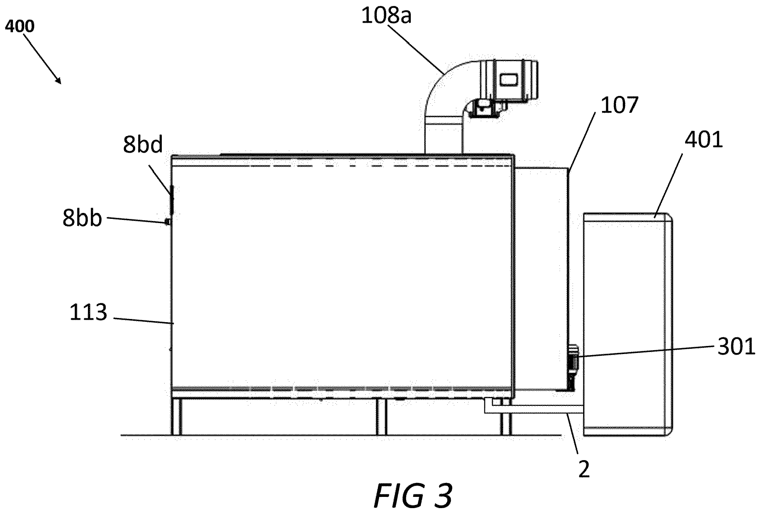

In Fig. 3 which is a side view of the apparatus 400, there is further indicated the electrical panel 107 that is adjacent to behind the exit antechamber 103 or the entrance antechamber, and there is also indicated the ozone generation system 401 which has the form of a cupboard connected with the tubing system 2 (wired electrical connections are not shown in the figure).

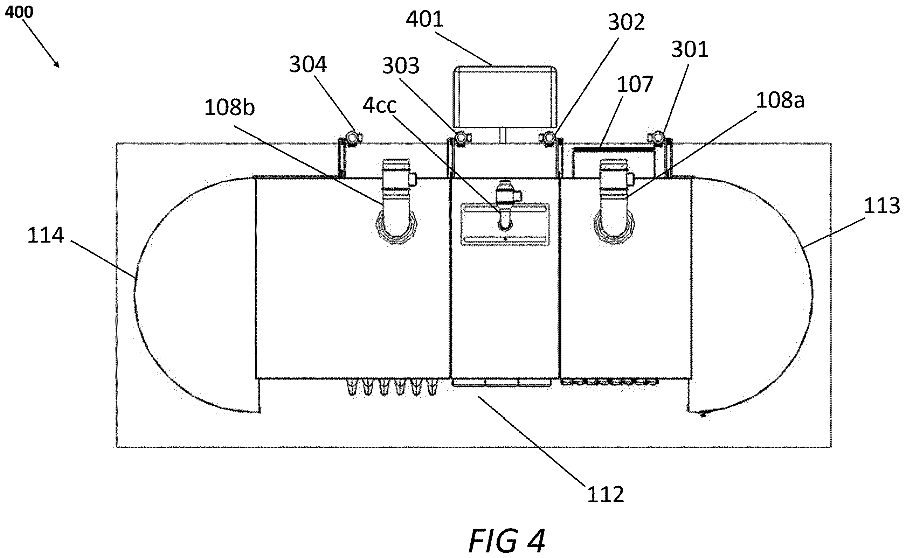

In Fig 4 which is a top view of the apparatus from above the ground, there are indicated the aforementioned exit door actuation mechanism 303, the entrance door actuation mechanism 302, the external exit door actuation mechanism 304, the external entrance door actuation mechanism 301, and each of said actuation mechanisms comprises a motor.

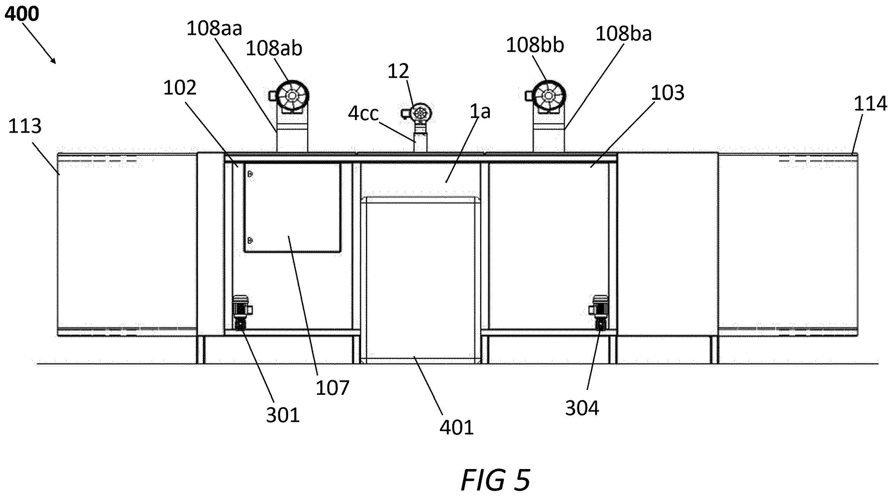

In Fig 5, which is a back view (view from behind the apparatus) there is further indicated that the first gas extractor comprises a first electric fan 108ab in the first extraction tube 108aa, and similarly the second gas extractor comprises a second electric fan 108bb in the second extraction tube 108ba. Similarly, in Fig 5 it is shown that the suction pump 12, which in this case is an electric fan, is installed within the upper part of the exhaust tube 4cc.

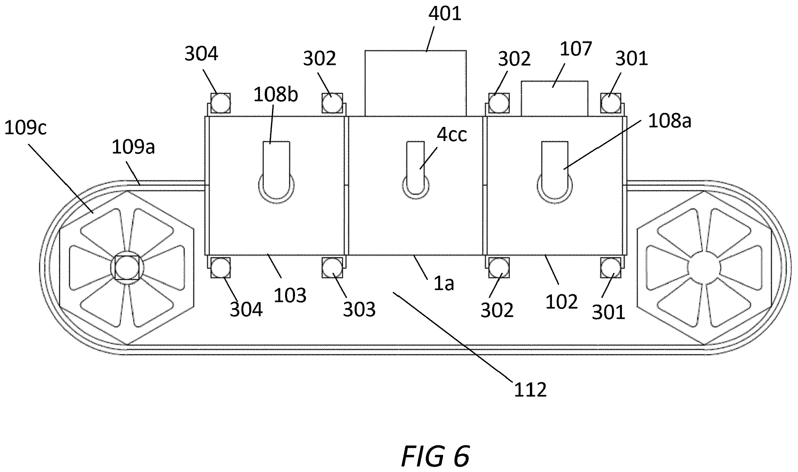

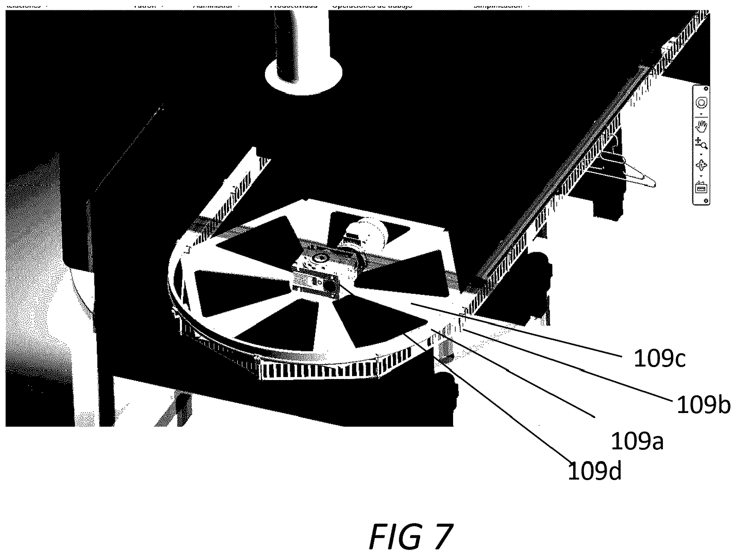

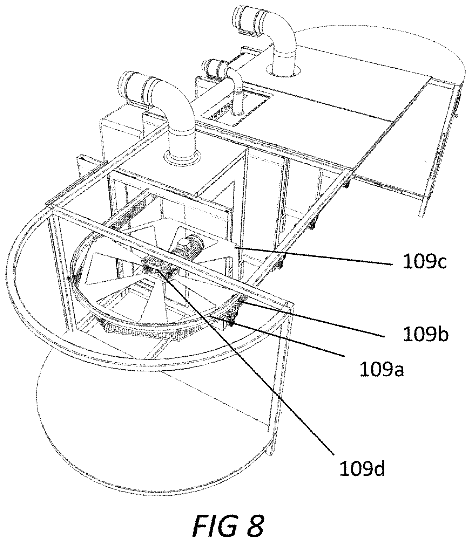

In FIG 6 there is another top view, but in this case the first and the second lobby structures are not shown so that the loop of the conveyor system and belt therein is better seen. As shown in FIG 6 the conveyor system is a closed-loop conveyor system having a loop of a substantially discorectangle (obround) shape. Therefore, the conveyor belt 109a of the system is arranged across a close-loop path of said shape. The items loaded on the belt follow this path when moved around by the conveyor system. Moreover, indicated in FIG 6, FIG 7 and FIG 8 the conveyor belt can be moved by the conveyor rotor 109c which is a rotatable wheel that is connected to and is movable by an electric motor. The electric rotor 109c (the wheel), the conveyor belt 109a, the conveyor chain 109b that is attached to the conveyor belt 109a, and the electric motor 109d that moves the wheel when the conveyor system is activated are shown in FIG 7 and FIG 8. The wheel is hexagonal in the shown case, but it may also be circular or have polygonal shape or form.

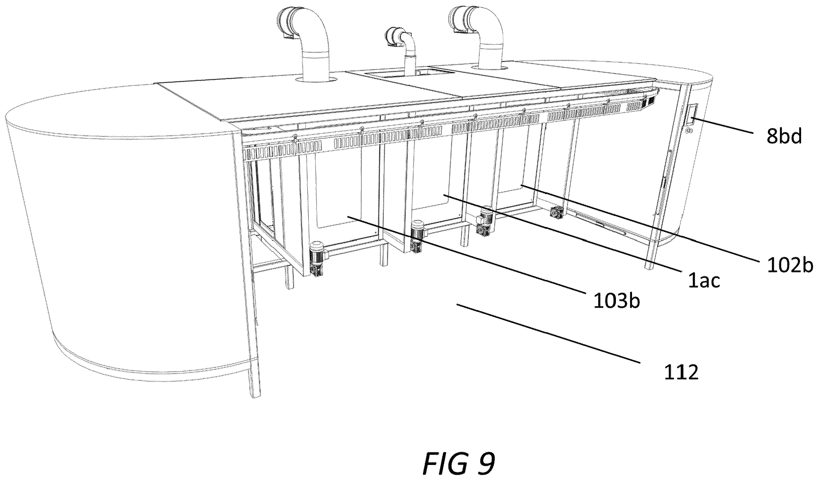

In the perspective of FIG 9 wherein part of the top ceiling of the apparatus has been omitted, there is seen that in the respective embodiment:
- the disinfection chamber has a middle viewing transparent window 1ac that allows viewing within the disinfection chamber from outside; and/or
- the entrance antechamber has a first viewing transparent window 102b that allows viewing within the entrance antechamber from outside; and/or
- the exit antechamber has a first viewing transparent window 103b that allows viewing within the exit antechamber from outside,
wherein said windows face the aforementioned loading and unloading area 112.

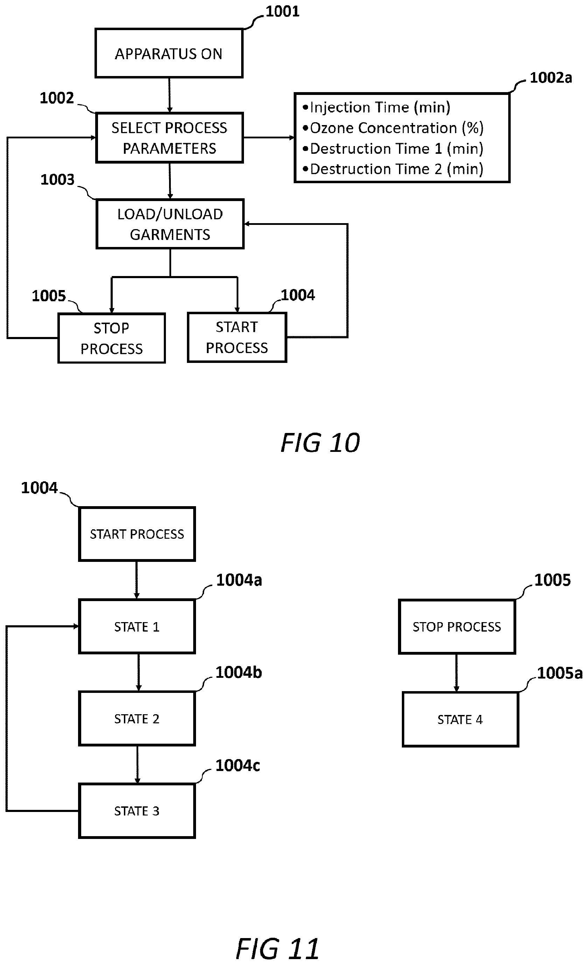

FIG 10 shows that sanitizing the garment using the apparatus may comprise:
- Turning ON 1001 the apparatus;
- Selecting 1002 process parameters;
- Loading/unloading 1003 garments on/from the apparatus;
- Starting 1004 the process;
- Stopping 1005 process and optionally further selecting 1002 parameters.

As indicated selecting 1002 parameters may comprise setting 1002a any of the following parameters:
- Injection Time (min), that may correspond to the duration of injecting ozone to the disinfection chamber;
- Ozone Concentration (%), that may correspond to a measured ozone concentration in gas;
- Destruction Time 1 (min), that may correspond to the duration of using the ozone destruction system with the exit door and entrance door closed;
- Destruction Time 2 (min), that may correspond to the duration of using the ozone destruction system and/or the gas extraction system with the exit door and entrance door open.

As indicated in FIG 10 when stopping 1005 the process, selecting 1002 parameters may be repeated, and when starting 1004 the process loading/unloading garments may be repeated.

As shown in FIG 11 after starting 1004 the process and related operation of the apparatus, there are 3 particular states, namely state 1 (first state) herein also denoted as 1004a, state 2 (second state) herein also denoted as 1004b, and state 3 (third state) herein also denoted as 1004c in which the apparatus may successively transition into during performing the process. Likewise, during the process an apparatus may go from state 3 to state 1. Likewise, when stopping 1005 the process the apparatus may transition into (assume) a state 4 (fourth state) that herein is also denoted as 1005a.

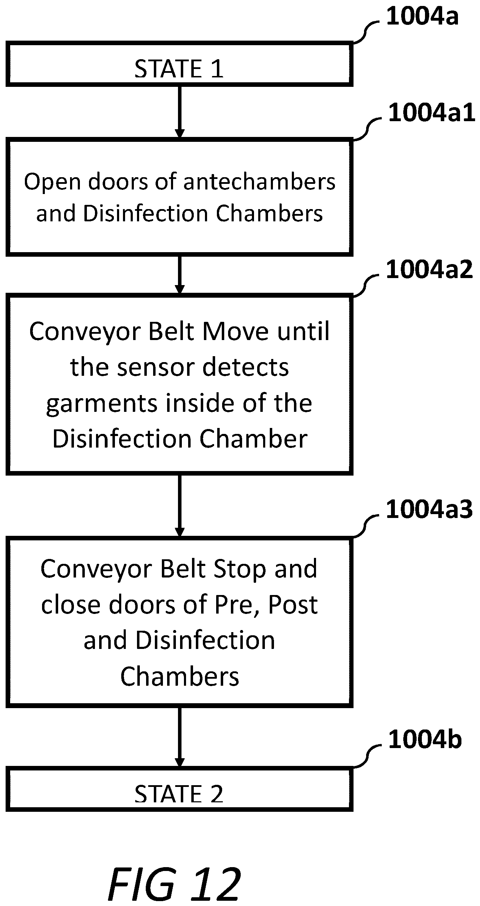

FIG 12 shows the actions related to transitioning from the first state 1004a to the second state 1004b, and said actions comprise:
- Opening 1004a1 the external entrance door, the external exit door, the exit door, the entrance door, i.e. opening the doors of the disinfection chamber and of the antechambers;
- Moving 1004a2 the conveyor belt until detecting, e.g. with a position sensor, items, such as for example garments, inside the disinfection chamber;
- Stopping 1004a3 the conveyor belt and closing the external entrance door, the external exit door, the exit door and the entrance door, i.e. closing opening the doors of the disinfection chamber and of the antechambers.

After the aforementioned actions, as shown in FIG 12 the apparatus enters into the second state 1004b.

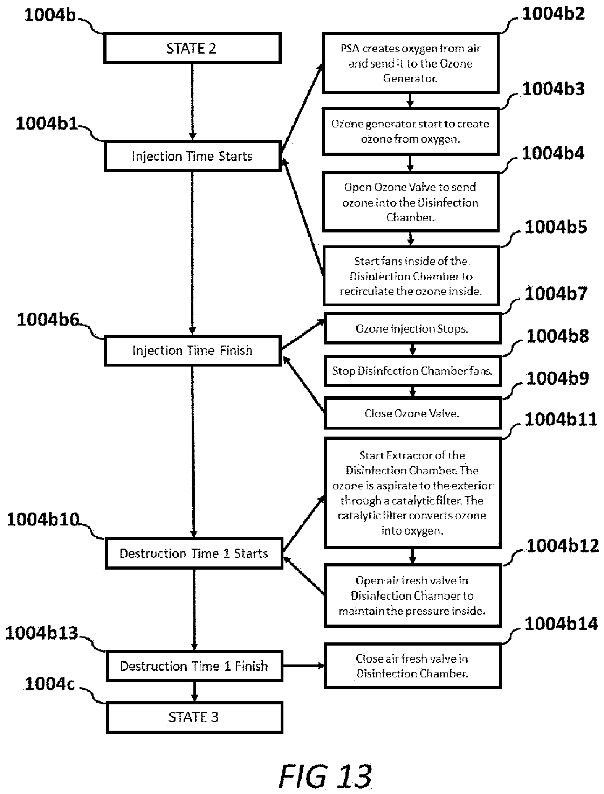

Likewise, FIG 13 shows the actions related to transitioning from the second state 1004b to the third state 1004c, and said actions comprise:
- starting 1004b1 injecting ozone (injection time starts);
- finishing 1004b6 injecting ozone (injection time finish);
- starting 1004b10 destructing ozone (destruction time 1 starts);
- finishing 1004b13 destructing ozone (destruction time 1 finish).

As shown in FIG 13, starting 1004b1 injecting ozone may comprise:
- using the ozone generator creating (separating) 1004b2 oxygen from air and sending the oxygen to the ozone generator;
- starting 1004b3 generating ozone from oxygen, using the ozone generator;
- sending 1004b4 ozone into the disinfection chamber, preferably by opening an ozone valve located in a part of the tubing system connecting the ozone generator and the disinfection chamber;
- starting 1004b5 ventilators (fans) inside the disinfection chamber to recirculate the ozone inside there.

Moreover, as shown in FIG 13, finishing 1004b6 injecting ozone (injection time finish) may comprise:
- stopping 1004b7 injecting ozone (ozone injection stops);
- stopping 1004b8 fans in disinfection chamber (stop disinfection chamber fans);
- closing 1004b9 ozone valve for further stopping injecting ozone in the disinfection chamber.

Moreover, as shown in FIG 13, starting 1004b10 destructing ozone (destruction time 1 starts) may comprise:
- starting extracting 1004b11 ozone from the disinfection chamber, preferably via starting 1004b11 the gas destruction system and extractor (suction pump) thereat (Start Extractor of the Disinfection Chamber. The ozone is aspirate to the exterior through a catalytic filter. The catalytic filter converts ozone into oxygen);
- opening 1004b12 an air vent valve connected to the disinfection chamber to allow air enter into the chamber to maintain the pressure thereat (Open air fresh valve in Disinfection Chamber to maintain the pressure inside).

It is noted that during starting extracting 1004b11 ozone, preferably the ozone is aspirate to the exterior through a catalytic filter, wherein the catalytic filter converts ozone into oxygen.

Moreover, as shown in FIG 13, starting finishing 1004b13 destructing ozone (destruction time 1 finish) may comprise:
- closing 1004b14 the air vent valve (air fresh valve) connected to the disinfection chamber.

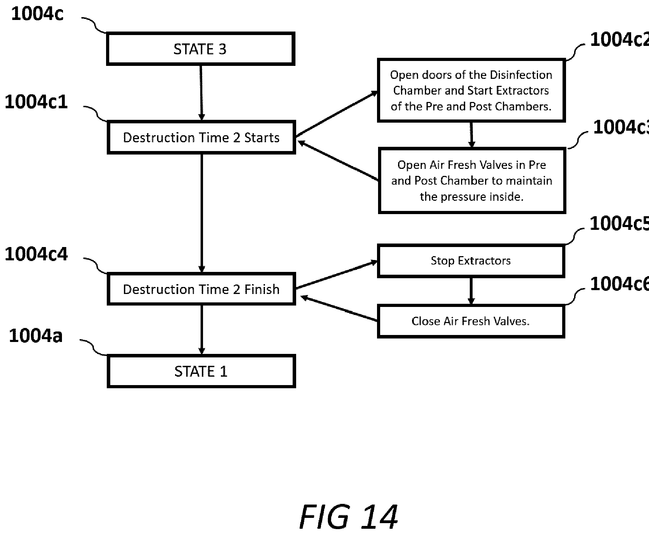

Likewise, FIG 14 shows the actions related to transitioning from the third state 1004c to the first state 1004a, and said actions comprise:
- Staring 1004c1 second destruction time (destruction time 2 starts);
- Finishing 1004c4 second destruction time (destruction time 2 finish).

Moreover, as shown in FIG 14, starting Staring 1004c1 second destruction time (destruction time 2 starts) may comprise:
- Opening 1004c2 the entrance door and the exit door and starting the extractors (the gas extraction system) of the entrance antechamber and the exit antechamber (Open doors of the Disinfection Chamber and Start Extractors of the Pre and Post Chambers);
- Opening 1004c3 the second air vent valve and the third air vent valve that are respectively connected to the entrance antechamber and the exit antechamber, for maintaining the pressure inside said antechambers (Open Air Fresh Valves in Pre and Post Chamber to maintain the pressure inside.)

Moreover, as shown in FIG 14, Finishing 1004c4 second destruction time (destruction time 2 finish) may comprise:
- stopping 1004c5 the gas extraction system (stopping the extractors);
- closing 1004c6 the second and the third air vent valves (close air fresh valves).

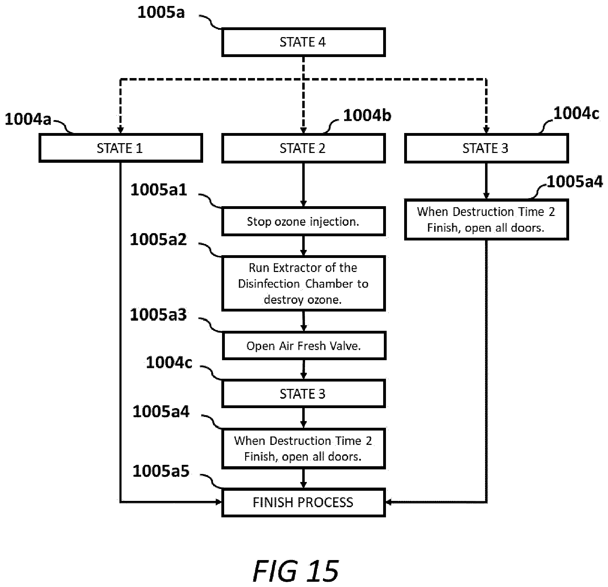

As shown in FIG 15, the apparatus may also be set at a fourth state 1005a (STATE 4) and from there it may be transitioned/set to the first state 1004a, or to the second state 1004b, or to the third state 1004c, and from thereon to the finish process 1005a5 state or moment.

Likewise, FIG 15 shows the actions related to transitioning from the second state 1004b towards the finish 1005a5 of the process via passing from third state 1004c, and said actions comprise:
- Stopping ozone injection 1005a1;
- Running/operating 1005a2 the ozone destroy system to destroy ozone from the disinfection chamber (Run Extractor of the Disinfection Chamber to destroy ozone.)
- Opening 1005c air vent valve (Open Air Fresh Valve.)
- Opening 1005a4 the exit door, the entrance door, the external exit door, the external entrance door, when the second destruction time finishes (When Destruction Time 2 Finish, open all doors.).

Likewise, FIG 15 shows transitioning from the third state 1004c towards the finish 1005a5 of the process, may comprise:
- Opening 1005a4 the exit door, the entrance door, the external exit door, the external entrance door, when the second destruction time finishes (When Destruction Time 2 Finish, open all doors.).

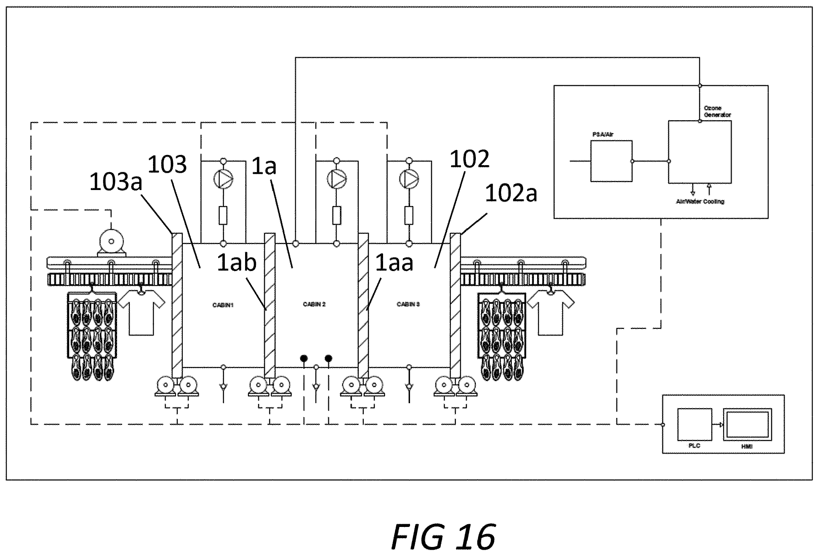

FIG 16, FIG 17 and FIG 18 essentially show the system shown in FIG 1.

FIG 16, shows that at the first state (state 1) all automatic doors (102a, 1aa, 1ab, 103a) are open, and no particular flow of gases is imparted within the apparatus by the systems therein

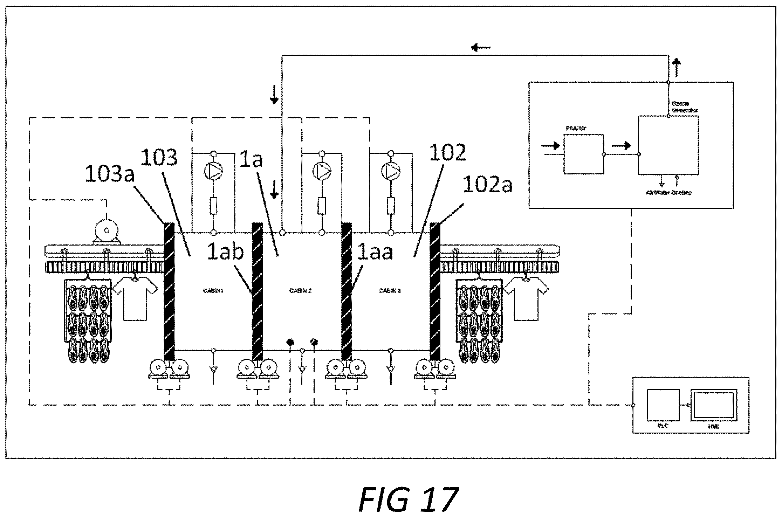

FIG 17 indicates that at the second state all the aforementioned automatic doors are closed, and the thick black arrows in FIG 17 shows that oxygen flows from the oxygen generator to the ozone generator, and ozone flows from the ozone generator to the disinfection chamber 1a.

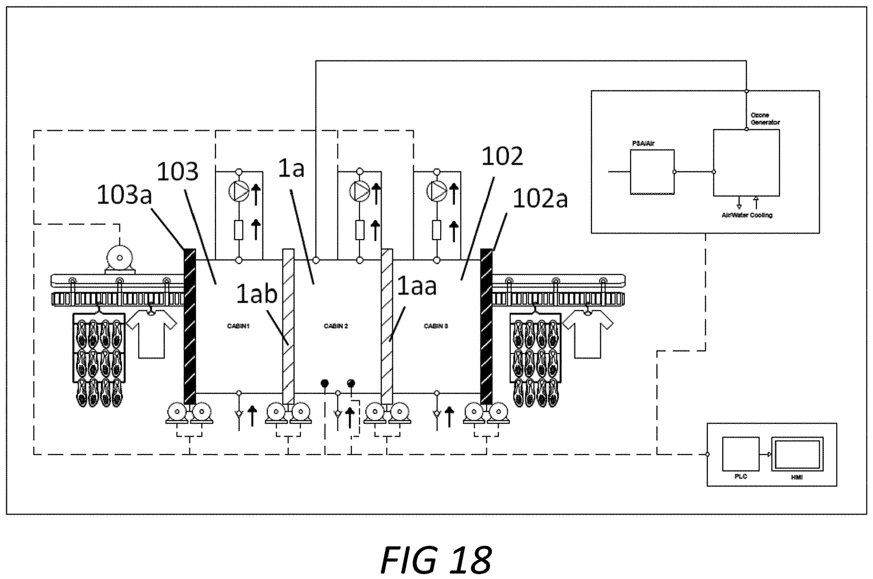

FIG 18 indicates that at the third stage entrance door and the external door are open, and the external entrance door and the external exit door are closed, and gases, such as ozone, flow from the disinfection chamber and the antechambers towards the ozone destruction system and towards the gas extractors, and also the air vent valves are open and air (fresh air) flows via said valves towards the interior of the disinfection chamber and the two antechambers.

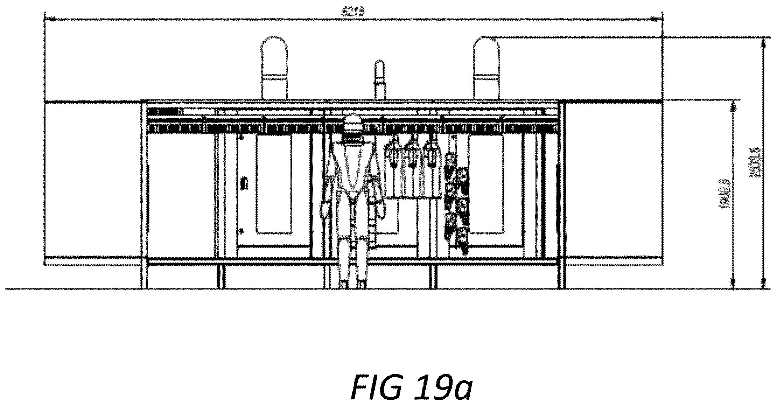

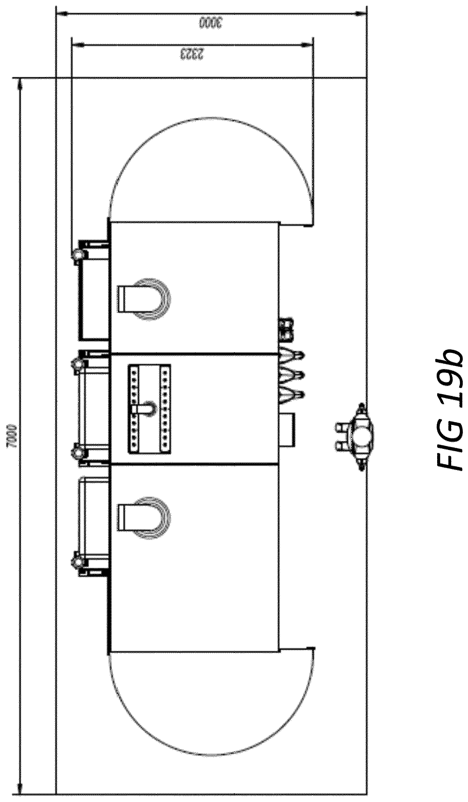

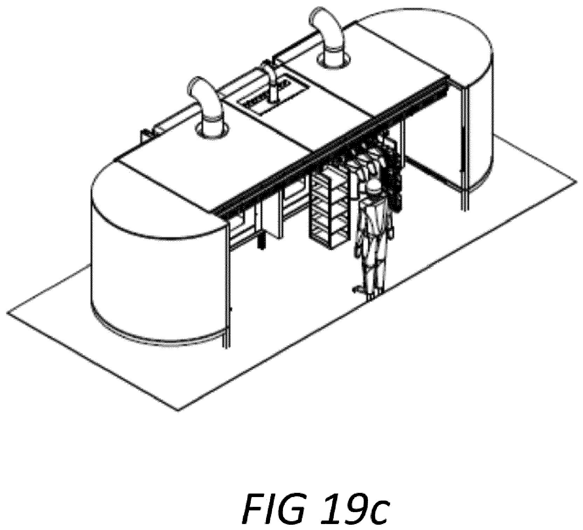

FIG 19a, FIG 19b and FIG 19C show different views of a system that is very similar in form and dimensions as the system shown in FIG 2, and FIG 19a, FIG 19b and FIG 19C indicate dimensions measured in millimetres (mm) and also include the figure of an adult so that the viewer can estimate the size of the apparatus. As shown the apparatus is exceptionally compact, while it can be used for processing in short times high volumes/numbers of items. A sanitizing cycle may actually last less than 15 minutes, or even less than 10 minutes, or even less than 5 min.

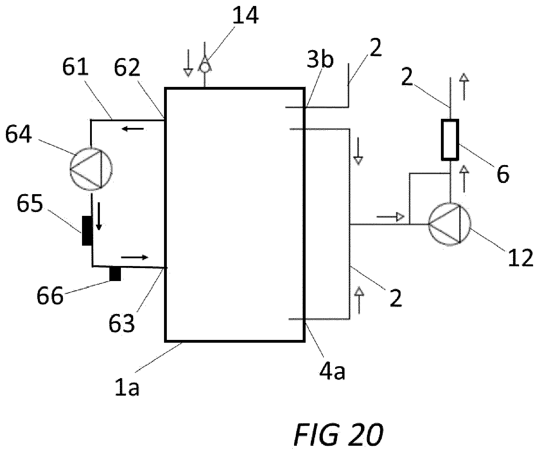

FIG 20 is a schematic diagram of a part of a preferred embodiment of an apparatus according to the first aspect of the invention, and said part includes the disinfection chamber 1a some of the apparatus' other features connected to said disinfection chamber. More specifically Fig 20 shows a case of an apparatus according to the first aspect of the invention, wherein the tubing system 2 comprises a tubing circuit 61 that has a circuit inlet 61 and a circuit outlet 63, said tubing circuit 61 being connected to the disinfection chamber 1a via the circuit inlet 62 and the circuit outlet 63, and the apparatus further comprises a heating system that when operated is configured to increase a temperature inside the disinfection chamber 1a, the heating system comprising a heating element 65, an auxiliary suction pump 64, and a temperature sensor 66, the heating element 65 when operated being configured to heat inside, i.e. heat any gases that flow via, the tubing circuit 61, the auxiliary suction pump 64 when operated being configured to force any gas from inside the disinfection chamber 1a to successively exit said disinfection chamber 1a via said circuit inlet 62, flow inside said tubing circuit 61, and reenter the disinfection chamber 1a via the circuit outlet 63. The direction of the flow of the gases inside the tubing circuit under the action of the auxiliary suction pump 64, is indicated by the black arrows thereat in Fig 20. In the system shown in Fig. 20 both the heating element and the temperature sensor are installed at the tubing circuit. For clarity of presentation certain features of the apparatus, e.g. the doors, are not shown in Fig. 20.

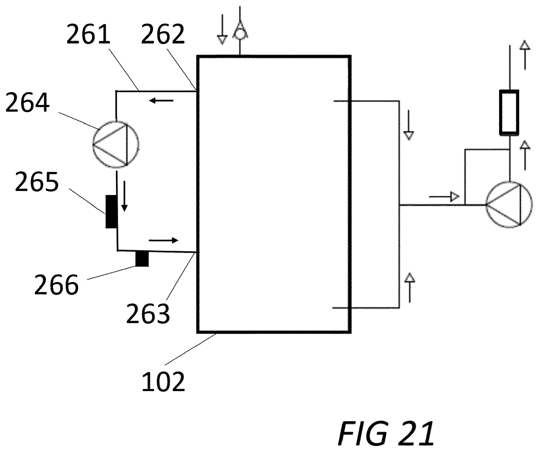

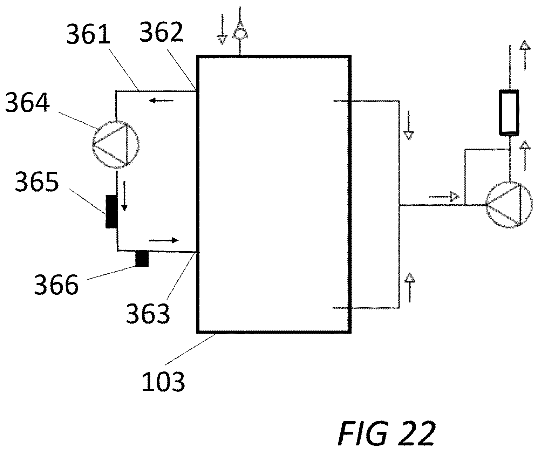

FIG 21 is a schematic diagram of a part of a preferred embodiment of an apparatus according to the first aspect of the invention, and said part includes the entrance antechamber 102 some of the apparatus' other features connected to said entrance antechamber 102. More specifically Fig 22 shows a case of an apparatus according to the first aspect of the invention, wherein the tubing system 2 comprises a second tubing circuit 261 that has a second circuit inlet 261 and a second circuit outlet 263, said second tubing circuit 261 being connected to the entrance antechamber via the second circuit inlet 262 and the second circuit outlet 263, and the apparatus further comprises a second heating system that when operated is configured to increase a temperature inside the entrance antechamber 102, the second heating system comprising a second heating element 265, a second auxiliary suction pump 264, and a second temperature sensor 266, the second heating element 265 when operated being configured to heat inside, i.e. heat any gases that flow via, the second tubing circuit s61, the second auxiliary suction pump 264 when operated being configured to force any gas from inside the entrance antechamber 102 to successively exit said entrance antechamber 102 via said second circuit inlet 262, flow inside said second tubing circuit 261, and reenter the entrance antechamber 102 via the second circuit outlet 263. The direction of the flow of the gases inside the second tubing circuit under the action of the second auxiliary suction pump 264, is indicated by the black arrows thereat in Fig 21. In the system shown in Fig. 21 both the second heating element and the second temperature sensor are installed at the second tubing circuit. For clarity of presentation certain features of the apparatus, e.g. the doors, are not shown in Fig. 21.FIG 22 is a schematic diagram of a part of a preferred embodiment of an apparatus according to the first aspect of the invention, and said part includes the exit antechamber 103 some of the apparatus' other features connected to said exit antechamber 103. More specifically Fig 22 shows a case of an apparatus according to the first aspect of the invention, wherein the tubing system 2 comprises a third tubing circuit 361 that has a third circuit inlet 361 and a third circuit outlet 363, said third tubing circuit 361 being connected to the exit antechamber via the third circuit inlet 362 and the third circuit outlet 363, and the apparatus further comprises a third heating system that when operated is configured to increase a temperature inside the exit antechamber 103, the third heating system comprising a third heating element 365, a third auxiliary suction pump 364, and a third temperature sensor 366, the third heating element 365 when operated being configured to heat inside, i.e. heat any gases that flow via, the third tubing circuit s61, the third auxiliary suction pump 364 when operated being configured to force any gas from inside the exit antechamber 103 to successively exit said exit antechamber 103 via said third circuit inlet 362, flow inside said third tubing circuit 361, and reenter the exit antechamber 103 via the third circuit outlet 363. The direction of the flow of the gases inside the third tubing circuit under the action of the third auxiliary suction pump 364, is indicated by the black arrows thereat in Fig 22. In the system shown in Fig. 22 both the third heating element and the third temperature sensor are installed at the third tubing circuit. For clarity of presentation certain features of the apparatus, e.g. the doors, are not shown in Fig. 22.

A preferred embodiment of the second aspect of the invention is a method that comprises using the preferred embodiment of the first aspect of the invention, and apply a process as described by FIG 11 and FIG 12, wherein said preferred embodiment of the method comprises all the steps described by FIG 13-15.

A preferred embodiment of the third aspect of the invention comprises instructions for executing the steps/actions of the method of the second aspect of the invention. All actions and processes described herein related to the operation of the apparatus, may be considered as corresponding parts of embodiments of the second and the third aspect of the invention.

While the foregoing is directed to embodiments of the present invention, other and further embodiments of the invention may be devised without departing from the basic scope thereof.

### CLAUSES

CLAUSE 1. An apparatus for sanitizing items, such as for example wearables, apparel or shoes, the apparatus comprising:
   - a disinfection chamber (1a) which has an entrance door (1aa) and an exit door (1ab) and is configured so that the items to be sanitized enter therein, meaning within it, via the entrance door (1aa), and exit therefrom, meaning from within the disinfection chamber (1a), via the exit door (1ab), the entrance door (1aa) and the exit door (1ab) being automatic;
   - a tubing system (2) that has an ozone inlet (3a), an ozone outlet (3b), an exhaust inlet (4a), an exhaust outlet (4b) and an end exhaust (4c), wherein the tubing system (2) is connected to the disinfection chamber (1a) via the ozone outlet (3b) and the exhaust inlet (4a), and is configured to, during an operation of the apparatus by a user, supply to the disinfection chamber (1a) ozone gas via the ozone outlet (3b), and to receive from the disinfection chamber (1a) ozone gas via the exhaust inlet (4a);

   - an entrance antechamber (102) that has an external entrance door (102a) and is adjacent to the disinfection chamber (1a) and to the entrance door (1aa) thereat, and is configured and arranged so that the items before entering the disinfection chamber (1a) pass via the external entrance door (102a) and through the entrance antechamber (102), the external entrance door (102a) being automatic;
   - an exit antechamber (103) that has an external exit door (103a) and is adjacent to the disinfection chamber (1a) and to the exit door (1ab) thereat, and is configured and arranged so that the items upon exiting the disinfection chamber (1a) pass through the exit antechamber (103) and exit therefrom via the external exit door (103a), the external exit door (103a) being automatic;
   - a motorized item transport system that is configured to convey the items so that, when the apparatus is operated, the items successively pass through the external entrance door (102a) and the entrance antechamber (102), enter and pass through the disinfection chamber (1a) and exit from the latter, and pass through the exit antechamber (103) exiting therefrom via the external exit door (103a);
   - an ozone generator (5) connected to the tubing system (2) and configured to generate ozone gas and supply via the ozone inlet (3a) said ozone gas to the tubing system (2) when the apparatus is operated, the ozone generator preferably being configured to generate ozone at a rate of between 1 grams of ozone per hour to 100 grams of ozone per hour, and preferably of between 50 grams of ozone per hour to 60 grams of ozone per hour and supplying said ozone gas to the disinfection chamber;
   - an ozone destroy system (6) connected to the tubing system (2) and is configured to receive from the latter via the exhaust outlet (4b) ozone gas that has been received by the tubing system (2) via the exhaust inlet (4a), and to convert said ozone gas to a non-toxic gaseous product, and to supply said non-toxic gaseous product to the end exhaust (4c);
   - a gas extraction system that is configured to extract gases from within the exit antechamber (103) and from the entrance antechamber (102) when the apparatus is operated;
   - sensing means (9) configured for sensing at least one operational parameter and for generating and transmit a corresponding sensing signal when the apparatus is operated;
   - a control and power system (7a) for controlling operating the apparatus, the control and power system (7a) comprising an electrical panel (107) for controlling powering the apparatus, a computer (8a) and user command means (8b) configured for receiving operation commands from a user, the computer preferably being a programmable logic controller, the user command means (8b) preferably comprising a touchscreen (8bd), and the control and power system (7a) is configured to control the operation of the apparatus according to the operation commands, and is configured to receive the sensing signal and inhibit the operation of the apparatus when the sensing signal does not correspond to a safety condition.
CLAUSE 2. An apparatus according to clause 1, wherein the motorized item transport system comprises or is a conveyor system (109) that extends through the disinfection chamber (1a), the entrance door (1aa), the entrance antechamber (102), the external entrance door (102a), the exit door (1ab), the exit antechamber (103) and the external exit door (103a).
CLAUSE 3. An apparatus according to clause 2, wherein the conveyor system (109) is a closed-loop conveyor system.
CLAUSE 4. An apparatus according to any of clauses 2-3, wherein the conveyor system extends over a loading and unloading area (112). The loading and unloading area (112) is designated for loading and unloading thereat items on the conveyor system, and preferably said area (112) is in front of any of the disinfection chamber (1a), the entrance antechamber (102) or the exit antechamber (103).
CLAUSE 5. An apparatus according to any of clauses 2-4, wherein the conveyor system comprises a conveyor belt (109a).
CLAUSE 6. An apparatus according to any of clauses 2-5, wherein the conveyor system comprises a conveyor chain (109b).
CLAUSE 7. An apparatus according to any of clauses 2-6, wherein the conveyor system comprises a hanging conveyor system that preferably comprises a conveyor belt (109a) and a conveyor chain (109b) that is attached to and hanging from said conveyor belt (109b), the conveyor chain (109b) having perforations adapted for hanging from the same said items, and/or for hanging hangers and/or containers and/or racks which are in turn adapted for placing on or hanging from the same the items.
CLAUSE 8. An apparatus according to any of clauses 2-7, wherein the conveyor system comprises or is a closed-loop hanging conveyor system that extends over a loading and unloading area (112) that is adapted for a user loading and unloading thereat items on the conveyor system.
CLAUSE 9. An apparatus according to any of clauses 2-8, wherein the conveyor system is adapted for hanging from the same the items to be sanitized and/or for hanging from the same hangers and/or racks and/or containers for loading thereon the items to be sanitized.
CLAUSE 10. An apparatus according to any of clauses 2-9, wherein the conveyor system (109) comprises an electric motor adapted for moving the conveyor.
CLAUSE 11. An apparatus according to any of clauses 2-10, wherein the conveyor system (109) comprises a rotor (109c) fitted with a respective electric motor (109d) that is controllable by the control and power system (7a) for rotating the rotor and moving the conveyor system and items thereon, wherein the rotor's shape preferably is polygonal, and more preferably is hexagonal.
CLAUSE 12. An apparatus according any of clauses 2-11, wherein the apparatus comprises a first lobby structure (113) that is adjacent to the entrance antechamber (102) and the external entrance door (102a) and is adapted so that the items on the conveyor system can pass through the first lobby structure (113) and along a first semicircular path, the second lobby structure preferably having a first semicylindrical shape.
CLAUSE 13. An apparatus according any of clauses 2-12, wherein apparatus comprises a second lobby structure (114) that is adjacent to the exit antechamber (103) and the external exit door (103a) thereat and is adapted so that the items can pass through the second lobby structure (114) along a second semicircular path, the second tunnel structure preferably having a second semicylindrical shape.
CLAUSE 14. An apparatus according to clauses 11 and 12, wherein the rotor (109c) is attached to a sealing of the first lobby structure (113).
CLAUSE 15. An apparatus according to clauses 11 and 13, wherein the rotor is attached to a sealing of the second lobby structure (114).
CLAUSE 16. An apparatus according to any of clauses 2-15, wherein the conveyor system (109) is a closed-loop conveyor system having a loop of a substantially discorectange (obround) shape.
CLAUSE 17. An apparatus according to clauses 12-16, wherein
   - the external entrance door (102a) and the entrance antechamber (102), the disinfection chamber (1a), the exit antechamber (103) and the external exit door (103a) are arranged in series along a first line segment of the discorectange shape;
   - the first lobby structure (113) and the first semicircular path are arranged along a first semicircle of the discorectange shape;
   - the second tunnel structure (114) and the second semicircular path are arranged along a second semicircle of the discorectange shape.
CLAUSE 18. An apparatus according to clauses 17 and 4, wherein the loading and unloading area (112) is arranged along a second line segment of the discorectange shape.
CLAUSE 19. An apparatus according to any of the preceding clauses, wherein the gas extraction system comprises a first gas extractor (108a) connected to the entrance antechamber (102) and configured for extracting from the latter gases, the first gas extractor preferably comprising a first extraction tube (108aa) and a first electric fan (108ab) therein, the first extraction tube (108aa) preferably projecting from a ceiling of and above the entrance antechamber (102).
CLAUSE 20. An apparatus according to any of the preceding clauses, wherein the gas extraction system comprises a second gas extractor (108b) connected to the exit antechamber (103) and configured for the extracting from the latter gases, the second gas extractor preferably comprising a second extraction tube (108ba) and a second electric fan (108bb) therein, the second extraction tube (108ba) preferably projecting from a ceiling of and above the exit antechamber (103).
CLAUSE 21. An apparatus according to any of the preceding clauses, wherein there is any of:
   - the ozone destroy system (6) and the gas extraction system are interconnected and configured so that when the apparatus is operated gases extracted with the gas extraction system are supplied to the ozone destroy system (6) wherein any ozone with said gases is converted to a non-toxic gaseous product; or
   - the gas extraction system comprises a second ozone destroy system that is configured to convert to a non-toxic gaseous product any gases extracted with the gas extraction system when the apparatus is operated.
CLAUSE 22. An apparatus according to any of the preceding clauses, wherein the entrance door (1aa), the exit door (1ab), the external entrance door (102a) and the external exit door (103a) are configured to close before or when the ozone generator (5) generates ozone gas, or when the ozone destruction system is operated, when the apparatus is operated.
CLAUSE 23. An apparatus according to any of the preceding clauses, wherein
   when or before the ozone destruction system is operated and/or when the gas extraction system is operated, the external entrance door (102a) and the external exit door (103a) are configured to be closed while the entrance door (1aa) and the exit door (1ab) are configured to be open.
CLAUSE 24. An apparatus according to any of the preceding clauses, wherein the entrance door (1aa) and/or the exit door (1ab) and/or the external entrance door (102a) and and/or the external exit door (103a) are configured to close non-hermetically.
CLAUSE 25. An apparatus according to any of the preceding clauses, wherein the motorized item transport system comprises a hanging conveyor system as is for example described in clauses 7 and 8, and wherein the entrance door (1aa) and/or the exit door (1ab) and/or the external entrance door (102a) and and/or the external exit door (103a) and/or have portions which are configured to contact or surround the hanging conveyor system when any of the entrance door (1aa) and/or the exit door (1ab) and/or the external entrance door (102a) and and/or the external exit door (103a) close, and any of said doors (1aa, 1ab, 102a, 103a) excluding said portions closes hermetically.
CLAUSE 26. An apparatus according to any of clauses 1-23, wherein the entrance door (1aa) and/or the exit door (1ab) and/or the external entrance door (102a) and and/or the external exit door (103a) are configured to close hermetically.
CLAUSE 27. An apparatus according to any of the preceding clauses, wherein:
   - the disinfection chamber has a middle viewing transparent window (1ac) that allows viewing within the disinfection chamber from outside; and/or
   - the entrance antechamber (102) has a first viewing transparent window (102b) that allows viewing within the entrance antechamber (102) from outside; and/or
   - the exit antechamber (103) has a first viewing transparent window (103b) that allows viewing within the exit antechamber (103) from outside,
   and preferably wherein any of the middle (1ac), first (102b) or second (103b) window faces a loading and unloading area (102).
CLAUSE 28. An apparatus according to any of the preceding clauses, wherein the apparatus comprises two ventilators located within the disinfection chamber, and preferably the two ventilators with respect to each other are located in opposite sides and more preferably on two opposite walls of the disinfection chamber, the ventilators being configured for forcing any gas within the disinfection chamber (1a) to move therein, each ventilator preferably being an electric fan.
CLAUSE 29. An apparatus according to any of the preceding clauses, that comprises a compartment (1e) that is adjacent to and preferably behind the disinfection chamber (1a) and contains within it any or both of the ozone generator (5) and the ozone destroy means (6).
CLAUSE 30. An apparatus according to any of the preceding clauses, wherein the end exhaust (4c) is on an exhaust tube (4cc) that projects from above the disinfection chamber.
CLAUSE 31. An apparatus according to any of the preceding clauses, wherein the electrical panel (107) is adjacent to and preferably behind the exit antechamber (103) or the entrance antechamber (102).
CLAUSE 32. An apparatus according to any of the preceding clauses, wherein the interior of the disinfection chamber (1a) where the items are to be inserted is made of a material that preferably comprises any of an oxidation resistive material, a stainless steel, a chromium-nickel stainless steel, stainless steel 316, stainless steel doped with molybdenum and combinations therefrom.
CLAUSE 33. An apparatus according to any of the preceding clauses, wherein the interior of the disinfection chamber (1a) in which the items are to be inserted has a volume of between 100 liters (litres) and 10000 liters, and preferably of between 500 liters and 4000 liters, and most preferably of between 900 and 1500 liters.
CLAUSE 34. An apparatus according to any of the preceding clauses, wherein the ozone generator (5) comprises a precursor entrance (5a) via which a precursor gas enters the ozone generator (5) when the latter generates ozone, the precursor gas preferably comprising ambient air, and/or preferably wherein the ozone generator is preferably a corona discharge ozone generator.
CLAUSE 35. An apparatus according to clause 34, wherein the precursor entrance communicates with a precursor opening at the disinfection chamber (1a), and via said precursor opening the precursor gas enters within the main body (1) and within the ozone generator (5).
CLAUSE 36. An apparatus according to any of clauses 34-36, that further comprises an oxygen generator (10) that is configured to generate oxygen and supply said oxygen to the precursor entrance (5a), and said precursor gas comprises oxygen, the oxygen generator preferably comprising a zeolite molecular sieve (ZMS) configured to absorb nitrogen molecules from the air thusly producing high purity oxygen of preferred concentration of between 90% and 97%.
CLAUSE 37. An apparatus according to clause 36, wherein the control and power system (7a) is configured to control the oxygen generator (10).
CLAUSE 38. An apparatus according to any of clauses 36-37, wherein the oxygen generator is configured to generate oxygen gas at rate of between 0.1 litre of oxygen per minute (L/min) and 100 L/min, and preferably of between 1 L/min and 20 L/min, and more preferably of between 4 L/min and 12 L/min, and most preferably the oxygen generator is configured to generate oxygen gas at rate of 10 L/min.
CLAUSE 39. An apparatus according to any of the preceding clauses, wherein the power supply is configured to:
   - supply electrical power of between 1 kW and 50 kW, and preferably of between 5 kW and 30 kW, and most preferably 25 kW; and/or
   - consume between 5 kWh and 15 kWh of electrical energy when operated.
CLAUSE 40. An apparatus according to any of the preceding clauses, that comprises a 63 A circuit breaker.
CLAUSE 41. An apparatus according any of the preceding clauses, that comprises a cooling system configured to cool the ozone generator when the latter and the apparatus are operated, the cooling system preferably being a water cooling system or an air cooling system, and wherein the control and power system (7a) and the cooling system are preferably configured so that the former controls the latter.
CLAUSE 42. An apparatus according to any of the preceding clauses, that comprises an ultraviolet (UV) radiation source located preferably on or within the disinfection chamber (1a), wherein the control and power system (7a) is configured to control the operation of the UV radiation source, and the latter when operated is configured to generate and supply UV radiation within the disinfection chamber (1a).
CLAUSE 43. An apparatus according to clause 42, wherein the UV radiation comprises any of UV-A, UV-B and UV-C, and combinations thereof, and preferably UV-C.
CLAUSE 44. An apparatus according to any of the preceding clauses, wherein the disinfection chamber (1a) comprises a UV filter that is preferably installed on a transparent window (1ac), as for example according to clause 27, thereat and is configured to block and prevent UV-C and/or UV-B and/or UV-A radiation from passing through said transparent window (1ac), when the door is closed.
CLAUSE 45. An apparatus according to any of the preceding clauses, that comprises a suction pump (12), that is connected to the tubing system (2) and is configured to be controllable by the power and control system (7a) when the apparatus is operated, the power and control system (7a) being configured to control the suction pump (12), and wherein the suction pump (12) and the tubing system (2) are configured to do any of the following and combinations thereof when the system is operated:
   - force ozone gas and/or other gases, for example atmospheric air and/or oxygen, to move out of the disinfection chamber (1a) and through the exhaust inlet (4a) and through the exhaust outlet (4b) and towards and/or through the ozone destroy system (6);
   - evacuate, e.g. evacuate the gaseous atmosphere out of, the disinfection chamber (1a);
   - force atmospheric air and/or oxygen gas to move into and/or through the tubing system (2) or parts thereof and/or into the disinfection chamber (1a), preferably through the ozone outlet (3b);
   - force the non-toxic gaseous product to move out of the ozone destroy system (6) and/or out of the system and/or through the end exhaust (4c);
   - force atmospheric air and/or oxygen gas to move into and/or through the tubing system and out of the ozone destroy system (6);
   - force a precursor gas, such as for example the precursor referred to in clause 34, to enter the ozone generator (5).
CLAUSE 46. An apparatus according to any of the preceding clauses, wherein the sensing means (9) comprise a lock sensor configured to sense whether the entrance door (1aa) or the exit door (1ab) and/or the external entrance door (102a) and and/or the external exit door (103a) is closed or not, and the safety condition comprises or is that the corresponding door is closed.
CLAUSE 47. An apparatus according to any of the preceding clauses, wherein the sensing means comprise an external ozone sensor that is located outside the disinfection chamber (1a) and preferably outside the entrance antechamber (102) and the exit antechamber (103), and is configured to measure the concentration of ozone thereat, and the safety condition comprises or is that the said concentration of ozone measured by the external ozone sensor is less than a safe external concentration that preferably is of 0.05 ppm (parts per million).
CLAUSE 48. An apparatus according to any of the preceding clauses, wherein the sensing means comprise an internal ozone sensor that is located within the disinfection chamber (1a) or within the tubing system (2), and is configured to measure the concentration of ozone thereat, and preferably the safety condition is or comprises that the concentration of ozone measured by the internal ozone sensor is less than a maximum allowed internal concentration of preferably 1010 ppm or 400 ppm or 300 ppm or 200 ppm.
CLAUSE 49. An apparatus according to any of the preceding clauses wherein the sensing means are electronic and connected via wire to the power and control system (7a), and the sensing signal is an electrical signal transmitted via said wire, and the power and control system (7a) and the sensing means (9) are configured so that the power and control system (7a) can detect and/or process said electric signal and/or can control the sensing means (9).
CLAUSE 50. An apparatus according to any of the preceding clauses, wherein the ozone destroy system (6) comprises a catalytic ozone destructor (6b) comprising a catalyst wherein preferably the catalyst comprises manganese oxide and/or copper oxide.
CLAUSE 51. An apparatus according to any of the preceding clauses, wherein the ozone destroy system (6) comprises a deodorization system (6a), such as for example a deodorization filter, that is configured to deodorize or aromatize the non-toxic gaseous product.
CLAUSE 52. An apparatus according to any of the preceding clauses, that comprises a ventilation system (23) configured to homogenize the ozone gas concentration within the disinfection chamber when the system and the ozone generator are operated, wherein preferably the ventilation system comprises two ventilators located within the disinfection chamber, and preferably the two ventilators with respect to each other are located in opposite sides and more preferably on two opposite walls of the disinfection chamber (1a), the ventilators being configured for forcing any gas within the disinfection chamber (1a) to move therein, each ventilator preferably being an electric fan, the ventilation system (23) and the power and control system being configured so that the latter controls the former.
CLAUSE 53. An apparatus according to clause 52, wherein the ventilation system comprises an electric fan located in the disinfection chamber or in or connected to the tubing system, and is configured to increase the travel speed or flow of any gas, such as ozone, that enters via the ozone outlet or is within the disinfection chamber.
CLAUSE 54. An apparatus according to any of the preceding clauses wherein the user command means (8b) comprise a power button (8ba) or knob and the like configured for switching on or off the power supply.
CLAUSE 55. An apparatus according to any of the preceding clauses, wherein the user command means (8b) comprise an emergency button (8bb) configured for stopping all operation of the apparatus without interrupting the power supply of the same.
CLAUSE 56. An apparatus according to any of the preceding clauses, wherein the user command means (8b) comprise a reset button (8bc) configured for restarting operation of the apparatus or of the power and control system or of the computer therein in case of a failing, and preferably for security reasons said reset button has to be used by the user every time the device is switched on.
CLAUSE 57. An apparatus according to any of the preceding clauses, wherein the user command means (8b) which preferably comprise an electronic touchscreen (8bd) are configured to display to the user several operation command options and sense a touching or selection of any of said operation command options by the user, and the computer is configured to convert said touching or selection of any of said operation command options to a corresponding operation command that is pre-stored and/or programmed in the computer.
CLAUSE 58. An apparatus according to any of the preceding clauses, wherein there is any of the following and combination thereof and preferably all of:
   - the tubing system (2) comprises an air vent with an air vent valve (14) therein configured to let air enter the tubing system and/or the disinfection chamber when the air vent valve is open, wherein said air vent valve is configured to be controllable by the power and control system or is mechanical;
   - the entrance antechamber (102) comprises an air vent with a second air vent valve (14a) therein configured to let air from outside the apparatus to enter the entrance antechamber (102) when the second air vent valve is open, wherein said second air vent valve is configured to be controllable by the power and control system or is mechanical;
   - the exit antechamber (103) comprises an air vent with a third air vent valve (14b) therein configured to let air from outside the apparatus to enter the exit antechamber (103) when the third air vent valve is open, wherein said third air vent valve is configured to be controllable by the power and control system or is mechanical.
CLAUSE 59. An apparatus according to any of the preceding clauses, wherein the apparatus comprises a position sensor that is configured to sense the position of a part of the motorized transport system and/or of an item loaded on the transport system, and said position sensor is preferably configured to sense whether said part or item is located within the disinfection chamber, and to generate a respective position sensing signal when said part or item is within the disinfection chamber, and further preferably the position sensor is connected to the power and control system and is controllable by the latter, the power and control system being configured to receive said position sensing signal.
CLAUSE 60. An apparatus according to any of the preceding clauses and clause 37 and clause 41 and clause 45 and clause 48 and clause 59 and any of clauses 52-53, wherein one of said pre-stored and/or programmed in the computer operation commands comprises instructions to execute (perform) any of the following or combinations thereof or preferably all of:
   - activate and/or set in motion the mechanized transport system until the position signal is generated;
   - close the entrance door (1aa) and the exit door (1ab)) and the external entrance door (102a) and the external exit door (103a);
   - activate the oxygen generator (10) to generate oxygen;
   - activate the gas extraction system to extract gases from the entrance antechamber and the exit antechamber;
   - activate the ozone generator to generate ozone gas, preferably for an ozone generation duration of between 1 second and 10 minutes (min), or for a disinfection duration of between 1 minute (min) and 20 min, and preferably of between 2 min and 10 min, while preferably measuring with the internal ozone sensor an ozone concentration of between 1 ppm and 1000 ppm;
   - activate/operate the cooling system and using the same to cool the ozone generator while generating with the ozone generator ozone gas;
   - activate the ventilation system to homogenize the ozone gas concentration within the disinfection chamber, preferably while generating with the ozone generator ozone gas,;
   - deactivate or disconnect or shut down or stop operating the ozone generator to stop generating ozone, and optionally, deactivate or disconnect or shut down or stop operating the oxygen generator and/or the cooling system and/or the ventilation system;
   - activate/operate operating the suction pump to remove ozone from the disinfection chamber, and open the air vent valve when the latter is controllable by the power and control system;
   - activate/operate the ozone destroy system (6) thusly destroying ozone gas;
   - open the exit door and the entrance door;
   - deactivate/stop the ozone destruction system and/or the suction pump, preferably when the internal ozone sensor measures an ozone concentration of less than 0.05 ppm and/or when a safety period of time since stopping the ozone generator has passed;
   - open the external entrance door and the external exit door, and preferably deactivate the gas extraction system;
   - activate and/or set in in motion the mechanized transport system to move the items outside the disinfection chamber and preferably outside the entrance and the exit antechambers;
   - preferably, indicate to the user via the touchscreen that the operation command has been executed.
CLAUSE 61. An apparatus according to any of the preceding clauses, wherein the items are cloths.
CLAUSE 62. An apparatus according to any of the preceding clauses, wherein the items are any of clothes, apparel, clothing accessories, trousers, shoes, hair caps, personal protective equipment (PPE) such as protective clothing, helmets, goggles, face masks or other garments or equipment designed to protect the wearer's body from injury or infection, and combinations thereof.
CLAUSE 63. An apparatus according to any of the preceding clauses, that comprises any or both of:
   - a humidity sensor located within the disinfection chamber or attached to or within the tubing system and configured to measure thereat a humidity concentration and generate and transmit a corresponding humidity signal;
   - a humidity control system configured to control the humidity within the disinfection chamber by introducing humidity, or removing humidity from within the disinfection chamber;
   wherein preferably the power and control system is configured to control the humidity sensor and receive said humidity signal, and/or is configured to control the operation of the humidity control system, the latter being configured to be controlled by the power and control system, the power and control system being preferably configured to operate the humidity control system to cause that the humidity concentration measured by the humidity sensor acquires a preferred humidity concentration value, the preferred humidity concentration value preferably being of between 8% and 80%, and more preferably of between 40% and 70%, and most preferably of between 60% and 70%.
CLAUSE 64. An apparatus according to any of the preceding clauses, wherein the operation command includes specifying a type of item or wearable or characteristic therein such as a material of the wearable, and/or adjusting a recipe parameter according to said specifying item or wearable or characteristic, said recipe parameter being any of the following and combination thereof:
   - disinfection/sanitization time;
   - ozone generation rate;
   - preferred Humidity value or range;
   - maximum allowed internal concentration of ozone (as measured by an internal ozone concentration sensor);
   - ozone concentration value or value range as measured by an internal ozone sensor;
   - ozone generation duration;
   - safety period of time.
CLAUSE 65. An apparatus according to any of the preceding clauses wherein the operation command may comprise accessing a computer file according to which an initial condition is correlated with at least one value or value range of any of the recipe parameters described in clause 64 or combinations thereof, said condition being any of a specific material to be disinfected, or an item to be disinfected, or a volume or quantity of items to be disinfected, or a specific level of disinfection required, or a specific protocol of disinfection required, and wherein the operation command may further comprise controlling the system and components thereof as required, to realize, as part of implementing a disinfection cycle, the recipe parameter value or value range that correlates to the initial condition.
CLAUSE 66. An apparatus according to any of the preceding clauses, wherein said apparatus further comprises any of:
   - a heating system that is configured to increase the temperature inside the disinfection chamber (1a);
   - a second heating system that is configured to increase the temperature inside the entrance antechamber;
   - a third heating system that is configured to increase the temperature inside the exit antechamber,
   or combinations thereof
CLAUSE 67. An apparatus according to clause 66, wherein any of the heating system, the second heating system and the third heating system is connected to and configured to be controllable by the power and control system, and the latter is configured to control the heating system.
CLAUSE 68. An apparatus according to any of clauses 66-67, wherein there is any of the following:
   - the heating system comprises a heating element installed (located) inside the disinfection chamber (1a) or inside the tubing system (2);
   - the second heating system comprises a second heating element installed (located) inside the entrance antechamber or inside the tubing system (2);
   - the third heating system comprises a third heating element installed (located) inside the exit antechamber or inside the tubing system (2).
CLAUSE 69. An apparatus according to clause 68, wherein the heating element, or the second heating element, or the third heating element, comprises an electrical resistance that preferably is configured to operate at a nominal power in the range of 1kW to 10 kW, and more preferably in the range of 1 kW to 3 kW.
CLAUSE 70. An apparatus according to any of clauses 66-69, wherein the heating system comprises a temperature sensor that is configured to measure a temperature inside the disinfection chamber (1a) or inside the tubing system (2), and/or wherein the second heating system comprises a second temperature sensor that is configured to measure a temperature inside the entrance antechamber or inside the tubing system (2), and/or wherein the third heating system comprises a third temperature sensor that is configured to measure a temperature inside the exit antechamber or inside the tubing system (2)
CLAUSE 71. An apparatus according to any of clauses 66-70, wherein the heating system comprise an auxiliary suction pump that is configured to force any gas from inside the disinfection chamber (1a) to flow by the heating element and to subsequently flow inside the disinfection chamber (1a), and/or wherein
CLAUSE 72. An apparatus according to clauses 66-71, wherein the tubing system (2) comprises a tubing circuit, and wherein the heating element and the auxiliary pump are installed (located, connected to) at said tubing circuit, and the tubing system and heating system, when the latter is operated, are configured to extract (any) gas from the disinfection chamber via said auxiliary suction pump, pass said gas via the tubing circuit and by the heating element thereat, heat said gas with the heating element, and reintroduce the thusly heated gas into the disinfection chamber.
CLAUSE 73. An apparatus according to clause 72, wherein the tubing circuit comprises a tubing circuit inlet connected to the disinfection chamber, a tubing circuit outlet connected to the disinfection chamber, and the auxiliary suction pump when operated is configured to extract gas from the disinfection chamber via said tubing circuit inlet, and reintroduce the gas intro disinfection chamber via said tubing circuit outlet.
CLAUSE 74. An apparatus according to any of clauses 66-73, wherein the temperature inside the disinfection chamber is the temperature of a gaseous atmosphere inside said disinfection chamber or inside the tubing circuit connected to said disinfection chamber, or is the temperature of any part of said gaseous atmosphere, and further preferably is the temperature measured by the temperature sensor described in clause 70.
CLAUSE 75. An apparatus according to any of clauses 66-74, wherein the heating system is configured to increase the temperature inside the disinfection chamber to a desired disinfection temperature, said desired disinfection temperature preferably being of between 25°C and 100°C, and more preferably of between 40°C to 90°C, and most preferably of between 60°C and 90°C.
CLAUSE 76. An apparatus according to any of the preceding claims, wherein the tubing system comprises a tubing circuit that has a circuit inlet and a circuit outlet, said tubing circuit being connected to the disinfection chamber (1a) via the circuit inlet and the circuit outlet, and the apparatus comprises a heating system that when operated is configured to increase a temperature inside the disinfection chamber (1a), the heating system comprising a heating element, an auxiliary suction pump, and a temperature sensor, the heating element when operated being configured to heat inside (i.e. heat the interior of, or the inside of, or any gases that are within) the tubing circuit, the auxiliary suction pump when operated being configured to force any gas from inside the disinfection chamber (1a) to successively exit said disinfection chamber via said circuit inlet, flow inside/via said tubing circuit, and reenter the disinfection chamber via the circuit outlet.
CLAUSE 77. An apparatus according to any of the preceding claims, wherein the tubing system comprises a second tubing circuit that has a second circuit inlet and a second circuit outlet, said second tubing circuit being connected to the entrance antechamber via the second circuit inlet and the second circuit outlet, and the apparatus comprises a second heating system that when operated is configured to increase a temperature inside the second antechamber, the second heating system comprising a second heating element, a second auxiliary suction pump, and a second temperature sensor, the second heating element when operated being configured to heat inside (i.e. heat the interior of, or the inside of, or any gases that are within) the second tubing circuit, the second auxiliary suction pump when operated being configured to force any gas from inside the entrance antechamber to successively exit said entrance antechamber via said second circuit inlet, flow inside/via said second tubing circuit, and reenter the entrance antechamber via the second circuit outlet.
CLAUSE 78. An apparatus according to any of the preceding claims, wherein the tubing system comprises a third tubing circuit that has a third circuit inlet and a third circuit outlet, said third tubing circuit being connected to the exit antechamber via the third circuit inlet and the third circuit outlet, and the apparatus comprises a third heating system that when operated is configured to increase a temperature inside the third antechamber, the third heating system comprising a third heating element, a third auxiliary suction pump, and a third temperature sensor, the third heating element when operated being configured to heat inside (i.e. heat the interior of, or the inside of, or any gases that are within) the third tubing circuit, the third auxiliary suction pump when operated being configured to force any gas from inside the exit antechamber to successively exit said exit antechamber via said third circuit inlet, flow inside/via said third tubing circuit, and reenter the exit antechamber via the third circuit outlet.
CLAUSE 79. An apparatus according to clause 60 and any of clauses 66-78, wherein one of said pre-stored and/or programmed in the computer operation commands comprises instructions to execute (perform) any of the following or combinations thereof or preferably all of:
   - activating or powering the heating system, or activating any components of said heating system, such as for example activating the auxiliary suction pump and the heating element, for increasing the temperature inside the disinfection chamber to preferably a desired disinfection temperature;
   - deactivating or powering off the heating system, or deactivating or powering of (partially or completely) any components of said heating system, such as for example deactivating the auxiliary suction pump and the heating element;
   - activating or powering the second heating system, or activating any components of said second heating system, such as for example activating the second auxiliary suction pump and the second heating element, for increasing the temperature inside the entrance antechamber to preferably a desired second disinfection temperature;
   - deactivating or powering off the second heating system, or deactivating or powering of (partially or completely) any components of said second heating system, such as for example deactivating the second auxiliary suction pump and the second heating element;
   - activating or powering the third heating system, or activating any components of said third heating system, such as for example activating the third auxiliary suction pump and the third heating element, for increasing the temperature inside the exit antechamber to preferably a desired third disinfection temperature;
   - deactivating or powering off the third heating system, or deactivating or powering of (partially or completely) any components of said third heating system, such as for example deactivating the third auxiliary suction pump and the third heating element.
CLAUSE 80. An apparatus according to clause 64 and any of clauses 66-79, wherein said recipe parameter being any of the following and combination thereof:
   - disinfection/sanitization time;
   - ozone generation rate;
   - preferred Humidity value (preferred humidity concentration value) or range;
   - maximum allowed internal concentration of ozone (as measured by an internal ozone concentration sensor);
   - ozone concentration value or value range as measured by an internal ozone sensor;
   - ozone generation duration;
   - total duration (of generating ozone with the ozone generation)
   - the desired disinfection temperature;
   - the desired second disinfection temperature;
   - the desired third disinfection temperature;
   - a duration of operating the heating system;
   - a duration of operating the second heating system;
   - a duration of operating the third heating system
CLAUSE 81. An apparatus according to any of the preceding claims, wherein the operation command may comprise accessing a computer file according to which an initial condition is correlated with at least one value or value range of any of the recipe parameters described in clause 80 or combinations thereof, said condition being any of a specific material to be disinfected, or an item to be disinfected, or a volume or quantity of items to be disinfected, or a specific level of disinfection required, or a specific protocol of disinfection required, and wherein the operation command may further comprise controlling the system and components thereof as required, to realize, as part of implementing a disinfection cycle, the recipe parameter value or value range that correlates to the initial condition.
CLAUSE 82. An apparatus for sanitizing items, the apparatus comprising:
   - a disinfection chamber (1a) which has an entrance door (1aa) and an exit door (1ab) and is configured so that the items to be sanitized enter within it via the entrance door (1aa) and exit from the disinfection chamber (1a) via the exit door (1ab), the entrance door (1aa) and the exit door (1ab) being automatic;
   - an entrance antechamber (102) that has an external entrance door (102a) and is adjacent to the disinfection chamber (1a) and the entrance door (1aa) thereat, and is configured and arranged so that the items before entering the disinfection chamber (1a) pass via the external entrance door (102a) and through the entrance antechamber (102), the external entrance door (102a) being automatic;
   - an exit antechamber (103) that has an external exit door (103a) and is adjacent to the disinfection chamber (1a) and the exit door (1ab) thereat, and is configured and arranged so that the items upon exiting the disinfection chamber (1a) pass through the exit antechamber (103) and exit therefrom via the external exit door (103a);
   - a motorized item transport system that when activated when the apparatus is operated is configured to convey the items so that the items successively pass through the external entrance door (102a) and the entrance antechamber (102), enter and pass through the disinfection chamber (1a) and exit from the latter, and pass through the exit antechamber (103) and exit therefrom via the external exit door (103a);
   - an ozone generator (5) connected to the disinfection chamber (1a) and configured to generate ozone gas and supply the latter to within (the interior of) the disinfection chamber (1a) when the apparatus is operated;
   - an ozone destroy system (6) connected to the disinfection chamber (1a) and comprising an end exhaust (4c) and configured to receive from within (the interior of) the disinfection chamber (1a) ozone gas and to convert said ozone gas to a non-toxic gaseous product, and to preferably release said non-toxic gaseous product via the end exhaust (4c), when the apparatus is operated;
   - a gas extraction system that is configured to extract gases from within the exit antechamber (103) and the entrance antechamber (102) when the apparatus is operated;
   - sensing means (6) configured for sensing at least one operational parameter and generating and transmit a corresponding sensing signal when the apparatus is operated;
   - a control and power system (7a) for controlling operating the apparatus, the control and power system (7a) comprising an electrical panel (107) for controlling powering the apparatus, a computer (8a) and user command means (8b) configured for receiving operation commands from a user, the computer preferably being a programmable logic controller, the user command means (8b) preferably comprising a touchscreen (8bd), and the control and power system (7a) is configured to control the operation of the apparatus according to the operation commands, and is configured to receive the sensing signal and inhibit the operation of the apparatus when the sensing signal does not correspond to a safety condition.
CLAUSE 83. An apparatus according to clause 80, that comprises a heating system that is configured to increase the temperature inside the disinfection chamber (1a), and/or a second heating system that is configured to increase the temperature inside the entrance antechamber, and/or a third heating system that is configured to increase the temperature inside the exit antechamber
CLAUSE 84. An apparatus according to clause 83, wherein the heating system comprises a heating element attached to or inside the disinfection chamber or the tubing system.
CLAUSE 85. An apparatus according to any of clauses 83-84, wherein the heating system, and/or the second heating system, and/or the third heating system, is connected to and configured to be controlled by the power and control system, and the latter is configured to control the heating system, and/or the second heating system, and/or the third heating system or any components therein, for operating said heating systems.
CLAUSE 86. A method for sanitizing an item or items with an apparatus, wherein said apparatus comprises a conveyor system, a disinfection chamber that has an entrance door and an exit door, an entrance antechamber that is adjacent to said disinfection chamber and to the entrance door and has an external entrance door, an exit antechamber that is adjacent to the disinfection chamber and to the exit door and has an external exit door, an ozone generator, an ozone destroy system, a gas extraction system that comprises a first gas extractor connected to the entrance antechamber and a second gas extractor connected to the exit antechamber, a power and control unit, the apparatus preferably being according to any of the preceding claims, the method comprising:
   - placing the item on the conveyor system;
   - moving the conveyor system for moving the item (or items) to be disinfected and inserting said item in the disinfection chamber;
   - stopping the motion of the conveyor system;
   - closing the entrance and the exit doors and the external entrance door the external exit door;
   - activating the gas extraction system thusly extracting gases from the entrance and the exit antechambers;
   - activating the ozone gas generator thusly generating ozone gas, and supplying said ozone gas to the disinfection chamber for disinfecting the items;
   - deactivating the ozone gas generator;
   - activating the ozone destruction system for removing ozone gas from the disinfection chamber and converting said ozone gas to a non-toxic gaseous product;
   - opening the entrance and exit doors;
   - operating the gas extraction system for preferably a safety period of time before preferably deactivating the gas extraction system;
   - deactivating the ozone destruction system;
   - opening the external entrance door and the external exit door
   - moving the conveyor system for moving the item and removing said item (or items) from within the disinfection chamber;
   - removing the item from the conveyor system.
CLAUSE 87. A method according to clause 86, wherein the apparatus comprises a second heating heating system that is configured to increase the temperature inside the entrance antechamber when said second heating system is activated (operated), and the apparatus further comprises a third heating system that is configured to increase the temperature inside the exit antechamber when said third heating system is activated (operated),
   and the method comprises any and preferably both of:
   - activating or powering (partially or completely) the second heating system (or any components of said heating system, such as for example a second auxiliary suction pump and/or a second heating element) thusly increasing the temperature inside the entrance antechamber to preferably a desired second disinfection temperature;
   - deactivating or powering off (partially or completely) the second heating system (or any components of said heating system, such as for example a second auxiliary suction pump and/or a second heating element;
   - activating or powering (partially or completely) the third heating system (or any components of said heating system, such as for example a third auxiliary suction pump and/or a third heating element) thusly increasing the temperature inside the exit antechamber to preferably a desired third disinfection temperature;
   - deactivating or powering off (partially or completely) the third heating system (or any components of said heating system, such as for example a third auxiliary suction pump and/or a third heating element;
CLAUSE 88. A method according to clause 87 wherein activating and/or deactivating the second and third heating systems (or any components of said heating system) is being performed before or after activating the ozone gas generator.
CLAUSE 89. A method according to any of clauses 86-88, wherein the items preferably are any of clothes, apparel, clothing accessories, towels, home textiles, linens, towels, trousers, shoes, jackets, coats, dresses, hair caps, wearables, personal protective equipment (PPE) such as protective clothing, helmets, goggles, face masks or other garments or equipment designed to protect the wearer's body from injury or infection, and combinations thereof.
CLAUSE 90. A method according to any of clauses 86-89, wherein placing the items to be disinfected comprises:
   - placing the items in or on a receptacle or a hanger or a rack that in turn is placed on or is hanged from the conveyor system.
CLAUSE 91. A method according to clause 90, wherein removing the items comprises avoiding touching the items, and/or comprises packeting the items within a plastic foil or bag or wrapping the items in said plastic foil or bag, and/or comprises placing a sign on the items or on a packet containing them wherein said sign indicates that the items have been sanitized.
CLAUSE 92. A method according to any of clauses 90-91, wherein removing the items may optionally comprise avoiding touching the receptacle or hanger.
CLAUSE 93. A method according to any of clauses 86-92, wherein after removing the sanitized items from the conveyor system optionally the method comprises any of the following and combinations thereof:
   - placing the items or a receptacle or hanger having them within a second package that for example is a second parcel or a sealable plastic bag or a plastic wrap, while preferably avoiding touching the items or a receptacle containing them;
   - placing a sign on said second parcel indicating that the items are disinfected or sanitized.
CLAUSE 94. A method according to any of clauses 86-93, that further comprises wearing personal protective equipment while applying the method or parts thereof.
CLAUSE 95. A method according to any of clauses 86-94, wherein generating ozone gas using the ozone generator comprises generating ozone gas at a rate of between 1 grams of ozone per hour to 100 grams of ozone per hour, and preferably of between 50 grams of ozone per hour to 60 grams of ozone per hour and supplying said ozone gas to the disinfection chamber.
CLAUSE 96. A method according to any of clauses 86-95, wherein generating ozone gas using the ozone generator and supplying said ozone gas to the disinfection chamber is optionally performed for an ozone generation duration of between 1 second and 10 minutes (min) or preferably of between 30 seconds and 5 min, or for a disinfection (sanitization) duration of between 1 second (s) and 20 min, and preferably of between 30 seconds and 10 min.
CLAUSE 97. A method according to any of clauses 86-96, wherein the method comprises supplying said ozone gas to the disinfection chamber at an ozone concentration of between 1 ppm and 1000 ppm as measured by an ozone sensor thereat, e.g. using an internal ozone sensor located at the disinfection chamber or at a tubing system that connects the ozone generator and the disinfection chamber and via which the ozone gas is being supplied.
CLAUSE 98. A method according to any of clauses 86-97, wherein the method comprises stopping passing said ozone gas to the disinfection chamber, and comprises converting said ozone gas to a non-toxic gaseous product, using the ozone destruction system, until the ozone concentration in gas within the disinfection chamber as measured by the aforementioned internal ozone sensor is less than 0.05 ppm, or until a safety period of time has passed.
CLAUSE 99. A method according to any of clauses 86-98, wherein the method further comprises while supplying said ozone gas to the disinfection chamber controlling the humidity within the disinfection chamber by introducing humidity, or removing humidity from within the disinfection chamber.
CLAUSE 100. A method according to clause 99 wherein controlling the humidity comprises causing that the humidity concentration within the chamber has or acquires a preferred humidity concentration value which preferably is of between 8% and 80%, and more preferably of between 40% and 70%, and most preferably of between 60% and 70%.
CLAUSE 101. A method according to any of clauses 86-100, wherein the apparatus is within any of a healthcare facility, a hospital, a medical center, a public service building, a school, a factory, a store, a laundry shop, a goods storage facility, a food production or processing facility or open field, a logistics center, a facility wherein cloths or apparel are made or processed or stored, and the items are any of clothes, apparel, trousers, shoes, hair caps, personal protective equipment (PPE) such as protective clothing, helmets, goggles, face masks or other garments or equipment designed to protect a wearer's body from injury or infection, and combinations thereof.

The clauses belong to the specification (description) of the present application.

## Claims

1. An apparatus for sanitizing items, the apparatus comprising:
- a disinfection chamber (1a) which has an entrance door (1aa) and an exit door (1ab) and is configured so that the items to be sanitized enter therein, meaning within it, via the entrance door (1aa), and exit therefrom, meaning from within the disinfection chamber (1a), via the exit door (1ab), the entrance door (1aa) and the exit door (1ab) being automatic;
- a tubing system (2) that has an ozone inlet (3a), an ozone outlet (3b), an exhaust inlet (4a), an exhaust outlet (4b) and an end exhaust (4c), wherein the tubing system (2) is connected to the disinfection chamber (1a) via the ozone outlet (3b) and the exhaust inlet (4a), and is configured to, during an operation of the apparatus by a user, supply to the disinfection chamber (1a) ozone gas via the ozone outlet (3b), and to receive from the disinfection chamber (1a) ozone gas via the exhaust inlet (4a);
- an entrance antechamber (102) that has an external entrance door (102a) and is adjacent to the disinfection chamber (1a) and to the entrance door (1aa) thereat, and is configured and arranged so that the items before entering the disinfection chamber (1a) pass via the external entrance door (102a) and through the entrance antechamber (102), the external entrance door (102a) being automatic;
- an exit antechamber (103) that has an external exit door (103a) and is adjacent to the disinfection chamber (1a) and to the exit door (1ab) thereat, and is configured and arranged so that the items upon exiting the disinfection chamber (1a) pass through the exit antechamber (103) and exit therefrom via the external exit door (103a), the external exit door (103a) being automatic;
- a motorized item transport system that is configured to convey the items so that, when the apparatus is operated, the items successively pass through the external entrance door (102a) and the entrance antechamber (102), enter and pass through the disinfection chamber (1a) and exit from the latter, and pass through the exit antechamber (103) exiting therefrom via the external exit door (103a);
- an ozone generator (5) connected to the tubing system (2) and configured to generate ozone gas and supply via the ozone inlet (3a) said ozone gas to the tubing system (2) when the apparatus is operated, the ozone generator preferably being configured to generate ozone at a rate of between 1 grams of ozone per hour to 100 grams of ozone per hour, and preferably of between 50 grams of ozone per hour to 60 grams of ozone per hour and supplying said ozone gas to the disinfection chamber;
- an ozone destroy system (6) connected to the tubing system (2) and is configured to receive from the latter via the exhaust outlet (4b) ozone gas that has been received by the tubing system (2) via the exhaust inlet (4a), and to convert said ozone gas to a non-toxic gaseous product, and to supply said non-toxic gaseous product to the end exhaust (4c);
- a gas extraction system that is configured to extract gases from within the exit antechamber (103) and from the entrance antechamber (102) when the apparatus is operated;
- sensing means (9) configured for sensing at least one operational parameter and for generating and transmit a corresponding sensing signal when the apparatus is operated;
- a second heating system that is configured to increase the temperature inside the entrance antechamber (102);
- a third heating system that is configured to increase the temperature inside the exit antechamber (103);
- a control and power system (7a) for controlling operating the apparatus, the control and power system (7a) comprising an electrical panel (107) for controlling powering the apparatus, a computer (8a) and user command means (8b) configured for receiving operation commands from a user, the computer preferably being a programmable logic controller, the user command means (8b) preferably comprising a touchscreen (8bd), and the control and power system (7a) is configured to control the operation of the apparatus according to the operation commands, and is configured to receive the sensing signal and inhibit the operation of the apparatus when the sensing signal does not correspond to a safety condition.

2. An apparatus according to claim 1, wherein the motorized item transport system comprises a conveyor system (109) that extends through the disinfection chamber (1a), the entrance door (1aa), the entrance antechamber (102), the external entrance door (102a), the exit door (1ab), the exit antechamber (103) and the external exit door (103a).

3. An apparatus according to claim 2, wherein the conveyor system (109) comprises a hanging conveyor system that comprises a conveyor belt (109a) and a conveyor chain (109b) that is attached to and hanging from said conveyor belt (109b), the conveyor chain (109b) having perforations adapted for hanging from the same said items, and/or for hanging hangers and/or containers and/or racks which are in turn adapted for placing on or hanging from the same the items.

4. An apparatus according to any of claims 2-3, wherein the conveyor system (109) comprises a rotor (109c) fitted with a respective electric motor (109d) that is controllable by the control and power system (7a) for rotating the rotor and moving the conveyor system and items thereon.

5. An apparatus according to any of the preceding claims, wherein the gas extraction system comprises a second gas extractor (108b) connected to the exit antechamber (103) and configured for the extracting from the latter gases.

6. An apparatus according to any of the preceding claims, wherein the gas extraction system comprises a second ozone destroy system that is configured to convert to a non-toxic gaseous product any gases extracted with the gas extraction system when the apparatus is operated.

7. An apparatus according to any of the preceding claims, wherein the interior of the disinfection chamber (1a) where the items are to be inserted is made of a material that comprises any of an oxidation resistive material, a stainless steel, a chromium-nickel stainless steel, stainless steel 316, stainless steel doped with molybdenum and combinations therefrom.

8. An apparatus according to any of the preceding claims, wherein the interior of the disinfection chamber (1a) in which the items are to be inserted has a volume of between 100 liters and 10000 liters, and preferably of between 500 liters and 4000 liters, and most preferably of between 900 and 1500 liters.

9. An apparatus according to any of the preceding claims, wherein the ozone generator (5) comprises a precursor entrance (5a) via which a precursor gas enters the ozone generator (5) when the latter generates ozone, the precursor gas comprising oxygen, and the apparatus further comprises an oxygen generator (10) that is configured to generate oxygen and supply said oxygen to the precursor entrance (5a).

10. An apparatus according to any of the preceding claims, wherein the sensing means comprise an external ozone sensor that is located outside the disinfection chamber (1a) and is configured to measure the concentration of ozone thereat, and the safety condition comprises that the said concentration of ozone measured by the external ozone sensor is less than a safe external concentration of 0.05 ppm, and the sensing means comprise an internal ozone sensor that is located within the disinfection chamber (1a) or within the tubing system (2) and is configured to measure the concentration of ozone thereat.

11. An apparatus according to any of the preceding claims, that comprises:
- a humidity sensor located within the disinfection chamber or attached to or within the tubing system and configured to measure thereat a humidity concentration and generate and transmit a corresponding humidity signal, and
- a humidity control system configured to control the humidity within the disinfection chamber by introducing humidity, or removing humidity from within the disinfection chamber.

12. An apparatus according to any of the preceding claims, wherein the ozone destroy system (6) comprises a catalytic ozone destructor (6b) comprising a catalyst.

13. An apparatus according to any of the preceding claims, wherein the second heating system is configured to increase the temperature inside the entrance antechamber to a second desired disinfection temperature, said second desired disinfection temperature being of between 25°C and 100°C, and more preferably of between 40°C to 90°C, and most preferably of between 60°C and 90°C.

14. A method for sanitizing an item or items with an apparatus, wherein said apparatus comprises a conveyor system, a disinfection chamber that has an entrance door and an exit door, an entrance antechamber that is adjacent to said disinfection chamber and to the entrance door and has an external entrance door, an exit antechamber that is adjacent to the disinfection chamber and to the exit door and has an external exit door, an ozone generator, an ozone destroy system, a gas extraction system that comprises a first gas extractor connected to the entrance antechamber and a second gas extractor connected to the exit antechamber, a second heating system that is configured to increase the temperature inside the entrance antechamber, a third heating system that is configured to increase the temperature inside the exit antechamber, a power and control unit, the apparatus being according to any of the preceding claims, the method comprising:
- placing the item on the conveyor system;
- moving the conveyor system for moving the item (or items) to be disinfected and inserting said item in the disinfection chamber;
- stopping the motion of the conveyor system;
- closing the entrance and the exit doors and the external entrance door the external exit door;
- activating the gas extraction system thusly extracting gases from the entrance and the exit antechambers;
- activating the ozone gas generator thusly generating ozone gas, and supplying said ozone gas to the disinfection chamber for disinfecting the items;
- deactivating the ozone gas generator;
- activating the ozone destruction system for removing ozone gas from the disinfection chamber and converting said ozone gas to a non-toxic gaseous product;
- opening the entrance and exit doors;
- operating the gas extraction system for preferably a safety period of time before preferably deactivating the gas extraction system;
- deactivating the ozone destruction system;
- opening the external entrance door and the external exit door;
- moving the conveyor system for moving the item and removing said item (or items) from within the disinfection chamber;
- removing the item from the conveyor system.
- activating the second heating system for increasing the temperature inside the entrance antechamber to a desired second disinfection temperature;
- activating the third heating system for increasing the temperature inside the exit antechamber to a desired third disinfection temperature;
- deactivating the second heating system;
- deactivating the third heating system.

15. A method according to claim 14, wherein the items preferably are any of clothes, apparel, clothing accessories, towels, home textiles, linens, towels, trousers, shoes, jackets, coats, dresses, hair caps, wearables, personal protective equipment and combinations thereof.
